# EUROPEAN PATENT APPLICATION

(11) **EP 4 817 621 A1**
(43) Date of publication of application: **07.10.2026**
(21) Application number: 25382337.1
(22) Date of filing: 03.04.2025
(51) Int. Cl.: A61K 35/28, A61K 9/00, A61K 9/19, A61K 38/17, A61K 47/26, A61P 19/02, C12N 5/0775

(54) **FORMULATION FOR THE TREATMENT OF OSTEOARTHRITIS**

(71) Applicant: Bioexolab, S.L., 08021 Barcelona (ES)
(72) Inventor: GUTIÉRREZ CASTRO, Francisco Alejandro, E-08021 Barcelona (ES); TINTORÉ CAICEDO, Javier, E-08021 Barcelona (ES)
(74) Representative: ABG Intellectual Property Law, S.L.

(57) **Abstract**

The present invention relates to lyophilized formulations deriving from adipose tissue mesenchymal stem cells secretome for the treatment of osteoarthritis.

## Description

### Field of the invention

The invention is related to the field of medicine, more particularly to the field of osteoarthritis.

### Background of the invention

Osteoarthritis (OA), a prevalent degenerative joint disease, represents one of the most significant challenges in regenerative medicine. Characterized by the progressive degradation of hyaline cartilage-the smooth, frictionless tissue covering articular surfaces-OA results in pain, stiffness, and impaired joint function and impacts patients' quality of life. Additionally, pathological changes in the subchondral bone, including remodelling and sclerosis, further exacerbate disease progression and symptoms. Despite the high prevalence of OA, current therapeutic approaches primarily focus on symptom management, such as pain relief and functional improvement, rather than addressing the underlying tissue degeneration.

Mesenchymal stem cells (MSCs) have emerged as a promising therapeutic tool in regenerative medicine due to their multipotent differentiation capabilities, particularly in chondrocytes (cartilage cells) and osteoblasts (bone cells). Sourced from different tissues, MSCs also exert paracrine effects by releasing bioactive molecules that modulate inflammation and promote tissue repair. These effects are due to a collection of bioactive substances, including soluble proteins, free nucleic acids, lipids, and extracellular vehicles (EVs), which can be further categorised into exosomes, microvesicles (or microparticles), and apoptotic bodies. In particular, exosomes are of special interest to OA management by promoting chondrogenesis, stimulating chondrocyte proliferation, and reducing chondrocyte apoptosis.

Exosomes originate from endosomes, and thus, the exosomal lipid membranes are enriched with cholesterol, sphingomyelin, and ceramide, characteristic components of lipid rafts. Exosomes are primarily recognised as mediators of intercellular communication, transferring proteins, lipids, and nucleic acids between cells to elicit responses based on their molecular cargo. However, when exosomes were first discovered, they were considered cell debris and were not studied.

Recent advances in MSC-derived exosome processing techniques have further enhanced their potential for clinical application. Different methods are available and have emerged to store the exosomes. Cryopreservation is one of the most commonly used methods to preserve the vesicle concentration, size, morphology, and functional properties. However, this method has been shown to affect the vesicles, causing ruptures, aggregations and precipitations.

In view of this, there is a pressing need for novel regenerative strategies to restore cartilage and subchondral bone, particular ones which are effective at regenerating the underlying tissue.

### Summary of the invention

The inventors have investigated the regenerative potential of human adipose tissue-derived MSCs (hAD-MSC) secretome containing exosomes in a preclinical lamb model, chosen for its physiological similarity to human joints in size and structure. Surgically induced articular lesions in the lambs' hind limbs mimic the degenerative processes observed in OA, allowing for the evaluation of cartilage and subchondral bone regeneration. Specifically, the inventors have compared the efficacy of three MSC secretome processing methods-fresh, cryopreserved, and lyophilised-in promoting tissue repair. Through histological analyses using techniques such as Hematoxylin-Eosin, Van Gieson, and Safranin-O staining, the study assesses extracellular matrix quality, chondrocyte presence, and vascularization in the subchondral bone. The inventors found surprisingly that the secretome had an increased effect on repair of damaged cartilage and subchondral bone regeneration after lyophilization.

A lyophilized secretome offers advantages in terms of storage, transport, and accessibility compared to fresh or cryopreserved secretomes, preserving the vesicle's size, content and bioactivity.

A first aspect of the invention relates to a lyophilized formulation comprising:
a) an adipose tissue mesenchymal stems cell (MSC) secretome, said secretome comprising:
   (i) extracellular vesicle particles; and
   (ii) at least one protein selected from a group consisting of: extracellular matrix protein 2 (ECM2), Prolargin, Vitamin D-binding protein (DBP), Midkine and any combination thereof; more preferably a combination of extracellular matrix protein 2 (ECM2), Prolargin, Vitamin D-binding protein (DBP) and Midkine;
      and
b) a cryoprotectant, wherein said cryoprotectant is trehalose.

Another aspect relates to a pharmaceutical composition comprising the lyophilized formulation according to the invention and a pharmaceutically acceptable diluent.

A further aspect relates to the lyophilized formulation or the pharmaceutical composition according to the invention for use in medicine.

A further aspect relates to the lyophilized formulation or the pharmaceutical composition according to the invention for use in the prevention and/or treatment of a joint lesion.

A further aspect relates to the lyophilized formulation or the pharmaceutical composition according to the invention for use in the prevention and/or treatment of a degenerative joint disease, preferably osteoarthritis.

A final aspect relates to a kit comprising the lyophilized formulation according to the invention and a pharmaceutically acceptable diluent.

### Brief description of the figures

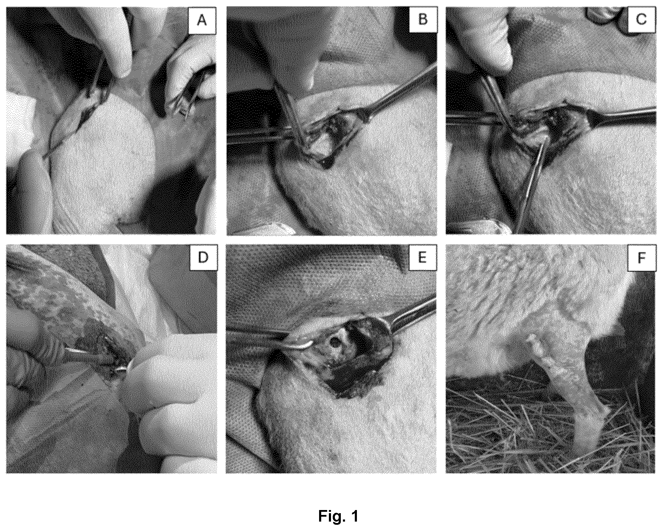

**Figure 1****. Animal Model of Osteoarthritis.** The surgical approach was performed laterally on the left hind limb, identifying the femoral trochlea using the lateral ligament as a reference (Images A-C). A 4 mm cartilage sample, including subchondral bone, was extracted with a biopsy punch (Images D-E) and preserved in buffered formalin for histopathological analysis. The joint capsule, muscle, and skin were sutured, and a sterile dressing was applied and maintained for 15 days (Image F).

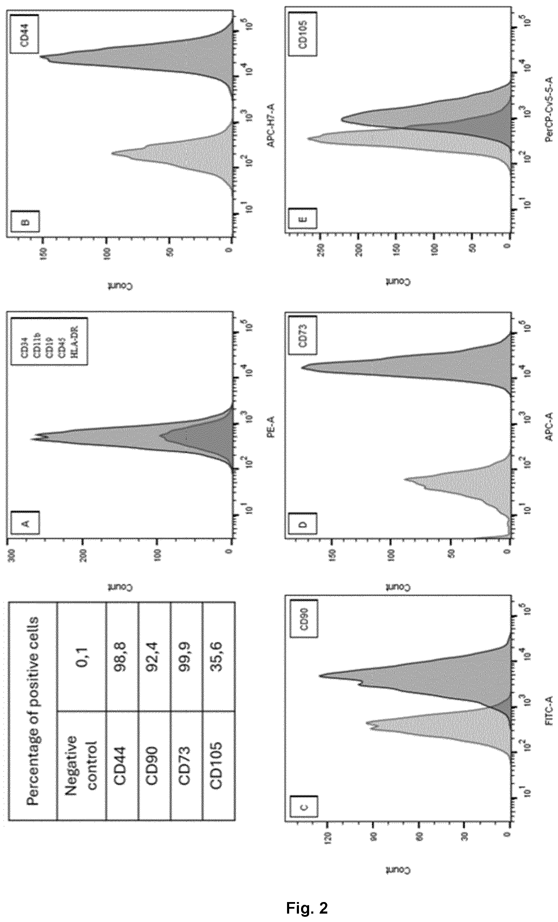

**Figure 2****. Analysis performed by flow cytometry on passage three cells (21 days of culture).** A: Histogram representing the negative selection cocktail composed of markers CD45, CD34, CD11b, CD19, and HLA-DR conjugated with phycoerythrin (PE). B: Histogram representing the expression of the marker CD44 conjugated with APCH7 (APC cyanine tandem dye). C: Histogram representing the expression of the marker CD90 conjugated with FITC (fluorescein isothiocyanate). D: Histogram representing the expression of the marker CD73 conjugated with APC (allophycocyanin). E: Histogram representing the expression of the marker CD105 conjugated with PerCP-CY 5.5 (phycoerythrin-cyanine 5.5).

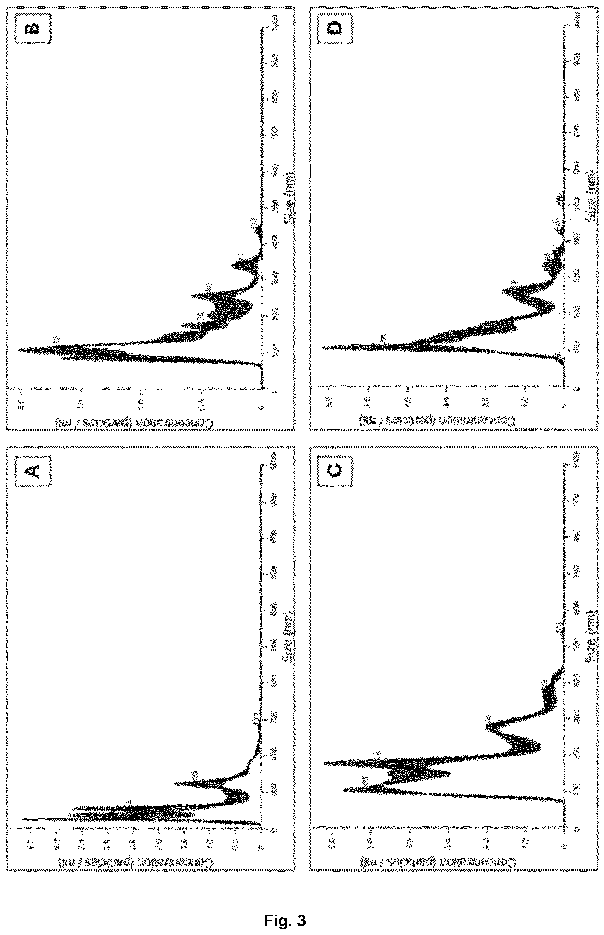

**Figure 3****. Quantification of extracellular vesicles (EVs) using Nanosight nanoparticle tracking analysis.** (A) The residual product from Vivaspin filtration inoculated in the control group derived from hAD-MSCs shows an average particle size of 75.9 ± 1.4 nm and a concentration of 1.78 × 10⁸ ± 4.29 × 10⁶ particles/ml. (B) The product applied to the fresh group shows an average particle size of 153.6 ± 12.5 nm and a concentration of 1.38 × 10⁹ ± 2.02 × 10⁸ particles/ml. (C) The product applied to the cryopreserved group shows an average particle size of 181.1 ± 3.1 nm and a concentration of 6.60 × 10⁹ ± 1.06 × 10⁸ particles/ml. (D) The product applied to the lyophilised group shows an average particle size of 167.6 ± 9.2 nm and a concentration of 3.70 × 10¹ ± 2.31 × 10⁸ particles/ml.

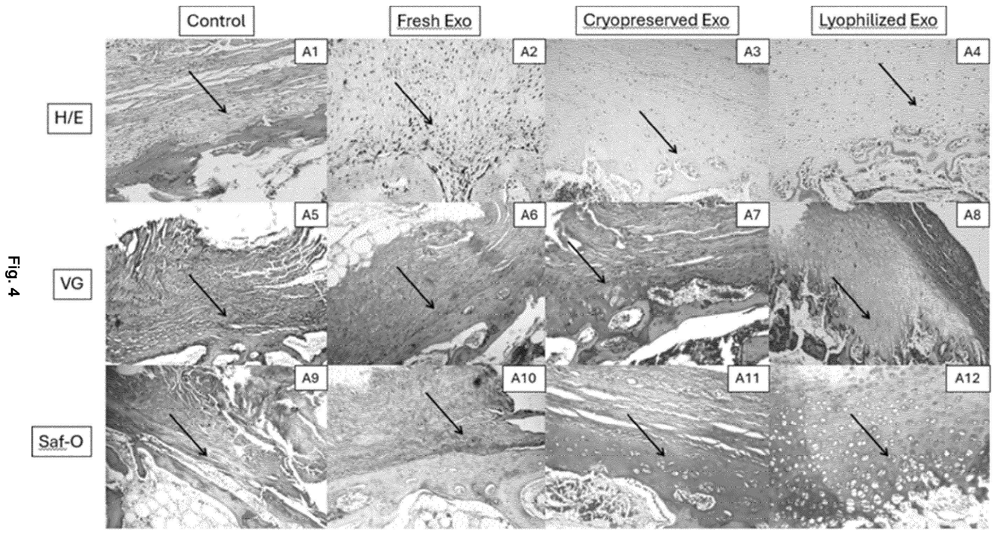

**Figure 4****. Cartilage regeneration analysis using histological staining.** Histological evaluations of cartilage regeneration were performed using three staining methods: hematoxylin-eosin (H/E) (A1-A4), Van Gieson's trichrome (VG) (A5-A8), and Safranin O (Saf-O) (A9-A12). Each column represents a specific experimental group: the control group (A1, A5, A9), the fresh group (A2, A6, A10), the cryopreserved group (A3, A7, A11), and the lyophilized group (A4, A8, A12). Arrows indicate the cartilage section within the tissue.

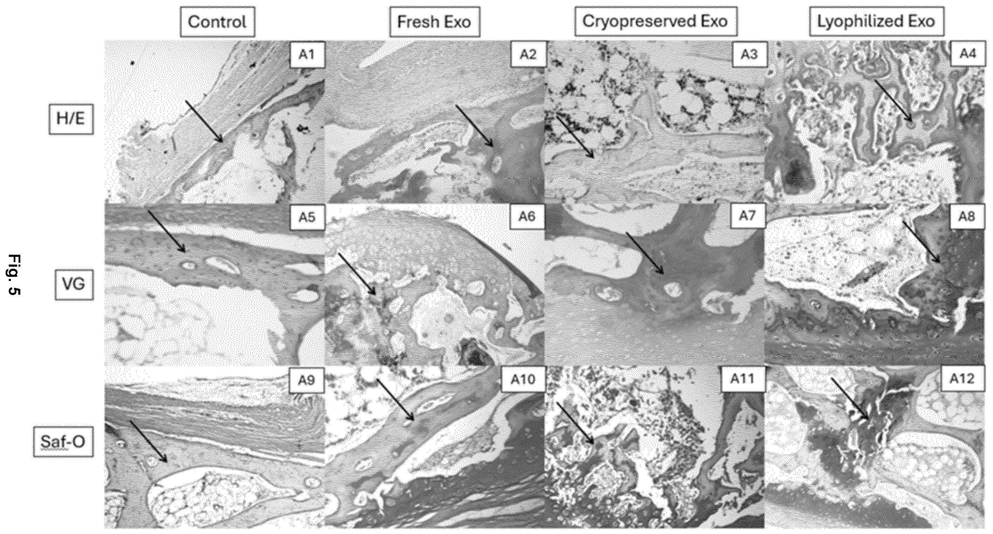

**Figure 5****. Subchondral Bone Regeneration.** Histological staining with hematoxylin-eosin (H/E) (A1-A4), Van Gieson's trichrome (VG) (A5-A8), and Safranin O (Saf-O) (A9-A12). Columns represent experimental groups: control (A1, A5, A9), fresh (A2, A6, A10), cryopreserved (A3, A7, A11), and lyophilized (A4, A8, A12). Arrows indicate the cartilage section within the tissue.

### Detailed description of the invention

A first aspect of the invention relates to a lyophilized adipose tissue mesenchymal stems cells secretome comprising extracellular vesicle particles, proteins secreted to the medium by the cells, and trehalose.

A first aspect of the invention relates to a lyophilized formulation comprising:
a) an adipose tissue mesenchymal stems cell (MSC) secretome, said secretome comprising:
   (i) extracellular vesicle particles; and
   (ii) at least one protein selected from a group consisting of: extracellular matrix protein 2 (ECM2), Prolargin, Vitamin D-binding protein (DBP), Midkine and any combination thereof; more preferably a combination of extracellular matrix protein 2 (ECM2), Prolargin, Vitamin D-binding protein (DBP) and Midkine; and
b) a cryoprotectant, wherein said cryoprotectant is trehalose.

As used herein, the term "lyophilized" relates to a formulation which has been subjected to the process of "lyophilization", also known as "lyophilizing," "freeze drying" or "cryodessication", which refers to a low temperature dehydration process that involves freezing a product and lowering pressure, removing the ice by sublimation. In a preferred embodiment the lyophilized secretome is a powder.

According to the invention the lyophilization is done to an adipose tissue mesenchymal stems cell secretome. The expression "adipose tissue mesenchymal stems cell secretome" refers to the fact that the secretome has been obtained from adipose tissue -derived mesenchymal stem cells. In a preferred embodiment the lyophilized adipose tissue mesenchymal stems cell secretome is obtained from adipose tissue-derived mesenchymal stems cells. In a preferred embodiment the adipose tissue MSC secretome is a mammalian adipose tissue MSC secretome, preferably is a human adipose tissue MSC secretome.

"Adipose tissue" (also known as body fat or simply fat) is a loose connective tissue composed mostly of adipocytes. The term "adipose tissue-derived cell" refers to a cell that originates from adipose tissue. The initial cell population isolated from adipose tissue is a heterogenous cell population including, but not limited to stromal vascular fraction (SVF) cells also known as adipose tissue-derived mesenchymal stromal cells, including preadipocytes, fibroblasts, vascular endothelial cells and a variety of immune cells such as adipose tissue macrophages, and adipose-derived stem cells (ASCs), also known as adipose tissue mesenchymal stem cells" (AMSCs). As used herein, the term "adipose tissue mesenchymal stem cells" (AMSCs) or simply mesenchymal stem cells (MSC) refers to stem cells that originate from adipose tissue which can serve as precursors to a variety of different cell types such as, but not limited to, adipocytes, osteocytes, and chondrocytes.

Adipose tissue-derived mesenchymal stromal cells and adipose tissue mesenchymal stem cells are obtained by methods well-known by the skilled person such as those disclosed in Example 1.

In a preferred embodiment the adipose tissue MSCs are characterized for having in their surface a marker selected from a group consisting of: CD44, CD90, CD73, and CD105, or any combination thereof. In another preferred embodiment the adipose tissue MSCs are characterized for having in their surface the markers CD44, CD90, CD73, and CD105. In a further preferred embodiment the adipose tissue MSCs are characterized for not having in their surface the markers CD45, CD34, CD11b, CD19, and HLA-DR.

As used herein, the term "marker" or "surface marker" refers to a protein, carbohydrate, lipid molecule, or a combination thereof, which is embedded in the cell membrane. These markers or marker combinations are unique to each cell type and allowing the identification and classification of different cell types. In the present invention, the markers allow the identification of adipose tissue MSCs.

As used herein, the term "secretome," also known as "conditioned medium", refers to the totality (or collection) of proteins (e.g., adhesion molecules, cytoskeletons, cytokines, ribosomal proteins, growth factors, and metabolic enzymes), lipids (including cholesterol, lipid rafts, and ceramides), nucleic acids (such as DNA, mRNA, and miRNA), metabolites, and even organelles, secreted from cells to the environment of the cells from which the secretome is obtained (into culture medium or the extracellular matrix surrounding the cells) when the cells are cultured or during the normal growth and development of the cells in their respective tissue. In a preferred embodiment, the lyophilized secretome comprises part or all of secreted proteins, secreted nucleic acid molecules, and secreted vesicles from the adipose derived mesenchymal stem cells.

The secretome of the invention can be obtained by culturing the adipose tissue mesenchymal stem cells isolated from the adipose tissue in a suitable medium, preferably DMEM-C. After a suitable incubation time, the medium contains the secretome, which is recovered as explained in Example 1.

As mentioned, the secretome includes vesicles or "particles" which are either secreted by the cells or are formed in the exterior of the cells by the materials secreted from the cells. In a preferred embodiment the particles have a size of up to 500 nm. In another embodiment the particles have an average particle size of between 155 nm and 180 nm, preferably an average particle size of 155 nm, 156 nm, 157 nm, 158 nm, 159 nm, 160 nm, 161 nm, 162 nm, 163 nm, 164 nm, 165 nm, 166 nm, 167 nm, 168 nm, 169 nm, 170 nm, 171 nm, 172 nm, 173 nm, 174 nm, 175 nm, 176 nm, 177 nm, 178 nm, 179 nm, 180 nm. In a preferred embodiment the average particle size is of 167.6 ± 9.2 nm. In another preferred embodiment the particles have a diameter of up to 100 nm, i.e., are nanoparticles.

The term "average particle size", also known as "mean particle size", as used herein refers to the overall average diameter of a population of particles in the secretome or a sample thereof, which is determined by calculating the diameters of every particle of a representative population of the particles of the secretome or sample thereof and averaging the results obtained.

The size of particles can be determined by several techniques well known to the skilled person in the art, such as, dynamic light scattering, disc centrifugation, particle tracking analysis, tunable resistive pulse sensing, atomic force microscopy, and electron microscopy. In an embodiment the particle size is determined by particle tracking analysis. In another embodiment the particle size is determined by light scattering and Brownian motion analysis.

In a preferred embodiment the particle size is determined at a temperature of 20°C to 30°C with a viscosity range of 0.7 - 1.2 cP, preferably at a temperature of 20°C, 21°C, 22°C, 23°C, 24°C, 25°C, 26°C, 27°C, 28°C, 29°C, or 30°C, with a viscosity 0.7 cP, 0.705 cP, 0.71 cP, 0.715 cP, 0.72 cP, 0.725 cP, 0.73 cP, 0.735 cP, 0.74 cP, 0.745 cP, 0.75 cP, 0.755 cP, 0.76 cP, 0.765 cP, 0.77 cP, 0.775 cP, 0.78 cP, 0.785 cP, 0.79 cP, 0.795 cP, 0.8 cP, 0.805 cP, 0.81 cP, 0.815 cP, 0.82 cP, 0.825 cP, 0.83 cP, 0.835 cP, 0.84 cP, 0.845 cP, 0.85 cP, 0.855 cP, 0.86 cP, 0.865 cP, 0.87 cP, 0.875 cP, 0.88 cP, 0.885 cP, 0.89 cP, 0.895 cP, 0.9 cP, 0.905 cP, 0.91 cP, 0.915 cP, 0.92 cP, 0.925 cP, 0.93 cP, 0.935 cP, 0.94 cP, 0.945 cP, 0.95 cP, 0.955 cP, 0.96 cP, 0.965 cP, 0.97 cP, 0.975 cP, 0.98 cP, 0.985 cP, 0.99 cP, 0.995 cP, 1 cP, 1.005 cP, 1.01 cP, 1.015 cP, 1.02 cP, 1.025 cP, 1.03 cP, 1.035 cP, 1.04 cP, 1.045 cP, 1.05 cP, 1.055 cP, 1.06 cP, 1.065 cP, 1.07 cP, 1.075 cP, 1.08 cP, 1.085 cP, 1.09 cP, 1.095 cP, 1.1 cP, 1.105 cP, 1.11 cP, 1.115 cP, 1.12 cP, 1.125 cP, 1.13 cP, 1.135 cP, 1.14 cP, 1.145 cP, 1.15 cP, 1.155 cP, 1.16 cP, 1.165 cP, 1.17 cP, 1.175 cP, 1.18 cP, 1.185 cP, 1.19 cP, 1.195 cP, or 1.2 cP, preferably at a temperature of 24°C with a viscosity range of 0.9-0.965 cP. In another preferred embodiment the particle size is determined at 25 frames per second with 60 second measurement time.

In an embodiment the particles which are comprised in the lyophilized adipose tissue mesenchymal stems cell secretome are extracellular vesicles.

As used herein, the term "extracellular vesicles (EVs)" refers to lipid bilayer-delimited particles that are naturally released from almost all types of cells but, unlike a cell, cannot replicate. EVs range in diameter from near the size of the smallest physically possible unilamellar liposome (around 20-30 nanometers) to as large as 10 microns or more, although the vast majority of EVs are smaller than 200 nm. EVs can be divided according to size and synthesis route into exosomes, microvesicles and apoptotic bodies. The composition of EVs varies depending on their parent cells, encompassing proteins (e.g., adhesion molecules, cytoskeletons, cytokines, ribosomal proteins, growth factors, and metabolic enzymes), lipids (including cholesterol, lipid rafts, and ceramides), nucleic acids (such as DNA, mRNA, and miRNA), metabolites, and even organelles. In an embodiment the extracellular vesicles are exosomes, microvesicles and/or apoptotic bodies.

The term "exosome", as used herein, refers to EVs ranging in size from 30 to 150 nanometers that are produced in the endosomal compartment of most eukaryotic cells. In a preferred embodiment the extracellular vesicles are exosomes.

The term "microvesicles", also known as "ectosomes" or "microparticles", as used herein, refers to EVs that are released from the cell membrane which can be as small as the smallest EVs (30 nm in diameter) or as large as 1000 nm. Preferably, microvesicles are larger than 150 nm, more preferably larger than 160 nm. Microvesicles play a role in intercellular communication and can transport molecules such as mRNA, miRNA, and proteins between cells. In a preferred embodiment the extracellular vesicles are microvesicles.

The term "apoptotic bodies", as used herein, refers to EVs which originate from cells having undergone apoptosis or programed cell death. In a preferred embodiment the extracellular vesicles are apoptotic bodies.

In an embodiment, the lyophilized adipose tissue mesenchymal stem cells secretome formulation according to the invention comprises particles, and at least a protein selected from the group consisting of: extracellular matrix protein 2 (ECM2), Prolargin, Vitamin D-binding protein (DBP) and Midkine. In another embodiment, the formulation comprises a combination of said proteins. Preferably said proteins are secretome proteins, more preferably not forming part of the extracellular vesicle particles, even more preferably forming part of the soluble fraction of the secretome.

In a preferred embodiment, the lyophilized adipose tissue mesenchymal stems cell secretome formulation according to the invention comprises particles, and the proteins extracellular matrix protein 2 (ECM2) Prolargin, Vitamin D-binding protein (DBP) and Midkine.

The term "Extracellular matrix protein 2" or its acronym "ECM2", is a protein encoded by the ECM2 gene, and plays a role in the assembly of elastic fibers within the extracellular matrix. Further details of ECM2 are available in the Uniprot database accession entry number O94769, version 187 of February 5, 2025. In an embodiment the formulation comprises Extracellular matrix protein 2.

The term "Prolargin", as used herein, refers to the protein encoded by the PRELP gene, and is a leucine-rich repeat protein present in connective tissue extracellular matrix. This protein functions as a molecule anchoring basement membranes to the underlying connective tissue. Further details of Prolargin are available in the Uniprot database accession entry number P51888, version 201 of February 5, 2025. In an embodiment the formulation comprises Prolargin.

The term "Vitamin D-binding protein" or its acronym "DBP", also known as gc-globulin (group-specific component), is a protein that in humans is encoded by the GC gene. DBP is genetically the oldest member of the albuminoid family and appeared early in the evolution of vertebrates. It is a multifunctional protein found in plasma, ascitic fluid, cerebrospinal fluid and on the surface of many cell types. Further details of DBP are available in the Uniprot database accession entry number P02774, version 241 of February 5, 2025. In an embodiment the formulation comprises Vitamin D-binding protein.

The term "Midkine" or its acronym "MDK", refers to a heparin-binding growth factor and cytokine, which plays a role in development, repair, and is upregulated during oncogenesis, inflammation, and tissue repair, potentially serving as a therapeutic target or biomarker for various diseases. Further details of MDK are available in the Uniprot database accession number P21741, version 241 of February 5, 2025. In an embodiment the formulation comprises Midkine.

In a preferred embodiment the secretome further comprises one or more of the proteins selected from the group consisting of: Secreted frizzled-related protein 4 (SFRP4), Tissue factor pathway inhibitor (TFPI), Adipocyte enhancer-binding protein 1 (AEBP1), Dickkopf-related protein 3 (DKK3), Plasminogen, and Endoglin. Preferably, the secretome further comprises Secreted frizzled-related protein 4 (SFRP4), Tissue factor pathway inhibitor (TFPI), Adipocyte enhancer-binding protein 1 (AEBP1), Dickkopf-related protein 3 (DKK3), Plasminogen, and Endoglin.

The term "Secreted frizzled-related protein 4" or its acronym "SFRP4", as used herein, refers to a protein encoded by the SFRP4 gene in humans, which is a member of the SFRP family that contains a cysteine-rich domain homologous to the putative Wnt-binding site of Frizzled proteins. SFRPs act as soluble modulators of Wnt signaling. Further details of SFRP4 are available in the Uniprot database accession entry number Q6FHJ7, version 179 of February 5, 2025. In an embodiment the formulation comprises Secreted frizzled-related protein 4.

The term "Tissue factor pathway inhibitor" or its acronym "TFPI", as used herein, refers to a single-chain polypeptide which can reversibly inhibit factor Xa (Xa). While Xa is inhibited, the Xa-TFPI complex can subsequently also inhibit the FVlla-tissue factor complex. TFPI contributes significantly to the inhibition of Xa *in vivo,* despite being present at concentrations of only 2.5 nM. The gene for TFPI is located on chromosome 2q31-q32.1, and has nine exons which span 70 kb. Further details of TFPI are available in the Uniprot database accession entry number P10646, version 255 of February 5, 2025. In an embodiment the formulation comprises Tissue factor pathway inhibitor.

The term "Adipocyte enhancer-binding protein 1" or its acronym "AEBP1", as used herein, refers to a transcriptional repressor that is down-regulated during adipogenesis, functions as a critical regulator of adipose tissue homeostasis through modulation of phosphatase and tensin homolog deleted on chromosome ten (PTEN) tumor suppressor activity and mitogen-activated protein kinase (MAPK) activation. Further details of AEBP1 are available in the Uniprot database accession entry number Q8IUX7, version 178 of February 5, 2025. In an embodiment the formulation comprises Adipocyte enhancer-binding protein 1.

The term "Dickkopf-related protein 3" or its acronym "DKK3", refers to a secreted glycoprotein that acts as a Wnt signaling pathway inhibitor, playing a role in development, stem cell differentiation, tissue homeostasis, and potentially serving as a biomarker and therapeutic target in various diseases, including cancer and kidney diseases. Further details of DKK3 are available in the Uniprot database accession entry number Q9UBP4, version 186 of February 5, 2025. In an embodiment the formulation comprises Dickkopf-related protein 3.

The term "Plasminogen", as used herein, refers to an enzyme (EC 3.4.21.7) present in blood that degrades many blood plasma proteins, including fibrin clots. The degradation of fibrin is termed fibrinolysis. In humans, the plasmin protein (in the zymogen form of plasminogen) is encoded by the PLG gene. Further details of Plasminogen are available in the Uniprot database accession entry number P00747, version 274 of February 5, 2025. In an embodiment the formulation comprises Plasminogen.

The term "Endoglin", also known as "CD105", as used herein, refers to a transmembrane glycoprotein primarily expressed on endothelial cells and plays a crucial role in angiogenesis, acting as a co-receptor for transforming growth factor-beta (TGF-β) ligands. Further details of Endoglin are available in the Uniprot database accession entry number P17813, version 235 of February 5, 2025. In an embodiment the formulation comprises Endoglin.

In a preferred embodiment the concentration of any one protein after reconstitution of the lyophilized formulation according to the invention is between 1 ng/mL to 100 µg/mL, between 1 ng/mL to 1000 ng/mL, between 50 ng/mL to 950 ng/mL, between 100 ng/mL to 900 ng/mL, between 150 ng/mL to 850 ng/mL, between 200 ng/mL to 800 ng/mL, between 250 ng/mL to 750 ng/mL, between 300 ng/mL to 700 ng/mL, between 350 ng/mL to 650 ng/mL, between 400 ng/mL to 600 ng/mL, between 450 ng/mL to 550 ng/mL, between 500 ng/mL to 550 ng/mL, between 1 µg/mL to 100 µg/mL, between 5 µg/mL to 95 µg/mL, between 10 µg/mL to 90 µg/mL, between 15 µg/mL to 85 µg/mL, between 20 µg/mL to 80 µg/mL, between 25 µg/mL to 75 µg/mL, between 30 µg/mL to 70 µg/mL, between 35 µg/mL to 65 µg/mL, between 40 µg/mL to 60 µg/mL, between 45 µg/mL to 55 µg/mL, or between 50 µg/mL to 55 µg/mL, preferably wherein the reconstitution is performed with 2 mL of a diluent.

In another preferred embodiment the concentration of all of the proteins after reconstitution of the lyophilized formulation according to the invention is of between 1 µg/mL to 100 µg/mL, between 1 ng/mL to 1000 ng/mL, between 50 ng/mL to 950 ng/mL, between 100 ng/mL to 900 ng/mL, between 150 ng/mL to 850 ng/mL, between 200 ng/mL to 800 ng/mL, between 250 ng/mL to 750 ng/mL, between 300 ng/mL to 700 ng/mL, between 350 ng/mL to 650 ng/mL, between 400 ng/mL to 600 ng/mL, between 450 ng/mL to 550 ng/mL, between 500 ng/mL to 550 ng/mL, between 1 µg/mL to 100 µg/mL, between 5 µg/mL to 95 µg/mL, between 10 µg/mL to 90 µg/mL, between 15 µg/mL to 85 µg/mL, between 20 µg/mL to 80 µg/mL, between 25 µg/mL to 75 µg/mL, between 30 µg/mL to 70 µg/mL, between 35 µg/mL to 65 µg/mL, between 40 µg/mL to 60 µg/mL, between 45 µg/mL to 55 µg/mL, or between 50 µg/mL to 55 µg/mL, preferably wherein the reconstitution is performed with 2 mL of a diluent.

In a preferred embodiment the secretome further comprises growth factors and cytokines.

In another embodiment the proteins present in the secretome of the lyophilized composition after reconstituting, are characterized by being up regulated by at least 0.5 fold in comparison with a fresh secretome, preferably wherein the reconstitution of the lyophilized formulation is done with 2 mL diluent.

In another embodiment the proteins present in the secretome of the lyophilized composition after reconstituting are characterized by being present in by an increase in quantity of at least 0.5 fold in comparison with a fresh secretome.

In another embodiment the secretome further comprises one or more of the proteins described in Table 4.

The term "up regulated", as used herein, refers to the fold change, preferably log fold change, between proteins of a fresh secretome and the proteins in the lyophilized formulation after being reconstituted, preferably with 2 - 10 mL of diluent, more preferably with 2 mL of diluent. The fold change is representative of a quantity, such as protein mass by mL (such as mg/mL) or representative of the number of peptides of each protein detected for each of the conditions when the quantification is made by mass spectrometry (MS).

As used herein, the term "cytokine" has the meaning normally ascribed to it in the art, i.e., a broad and loose category of small proteins (~5-25 kDa) important in cell signaling, which cannot cross the lipid bilayer of cells to enter the cytoplasm and therefore typically exert their functions by interacting with specific cytokine receptors on the target cell surface. Cytokines have been shown to be involved in autocrine, paracrine and endocrine signaling as immunomodulating agents. Cytokines include chemokines, interferons, interleukins, lymphokines, and tumour necrosis factors, but generally not hormones or growth factors, but herein it may also collectively refer to and include any growth factors. The term "growth factors", as used herein, refers to a naturally occurring substance capable of stimulating cell proliferation, wound healing, and occasionally cellular differentiation. Usually growth factors are secreted proteins or steroid hormones.

In some preferred embodiments the cytokine or the cytokine and/or growth factor may comprises any one or more of the following: FLT- 3L, INF-gamma, IL-IRA, IL-2, IL-3, IL-4, IL-5, IL-6, IL-7, IL-8, IL-10, IL-15, M-CSF, TGF- alpha, TNF-beta, Eotaxin, GRO-alpha, IL-12p70, IL-9, IL- 17 A, IL17F, MCP-3, MIP-1 -alpha, MIP-I-beta, VEGF-A, FGF-2, Fractalkine, IL-13, IP- 10, IL-22, TNF-alpha, G-CSF, IFN- alpha-2, IL-I-alpha, IL-12p40, IL-I-beta, EGF, PDGF-AA, MCP-1, IL-18, TGF-beta-2, sCD40L, IL-17E/IL-25, IL-27, MDC, MIG/CXCL9, PDGF-AB/BB, RANTES, TGF-beta-1, or any combination thereof.

The lyophilized formulation further contains trehalose. The term "trehalose", as used herein, refers to a naturally occurring, non-reducing disaccharide, comprising two glucopyranosyl units connected via an a, a-1, 1-glycosidic bond. Useful forms of trehalose can include trehalose dihydrate (TD) which is crystalline, amorphous trehalose (AT) which is a vitreous form, and the anhydrous forms of trehalose, anhydrous amorphous trehalose (AAT) and anhydrous crystalline trehalose (ACT). Powdered anhydrous trehalose may contain AAT and/or ACT. The term "trehalose," as used herein, refers to any physical form of trehalose including anhydrous, partially hydrated, fully hydrated and mixtures and solutions thereof.

Trehalose protects, moisturizes and has antioxidant properties. Trehalose is a naturally occurring bioprotective disaccharide that is present in numerous non-mammalian species, enabling survival in osmotically unfavorable environments by playing an important role in anhydrobiosis, desiccation and protection against oxidative and other stresses. It acts as a bioprotector which helps to maintain the integrity of cell membranes, protects proteins, promotes homeostasis, and activates autophagy, reducing cell death associated with apoptosis and inflammation. It has several medical applications, such as cryopreservation and organ transplantation or improving the stability of antibodies.

In a preferred embodiment the concentration of trehalose is of between 20 mM and 30 mM before lyophilization. In another preferred embodiment the concentration of trehalose is of 25 mM before lyophilization of the formulation.

As the expert in the art will understand the concentrations of a lyophilized formulation can only be determined either before the formulation is lyophilized or after the lyophilized formulation is reconstituted. In a preferred embodiment all the concentrations described for the components of the lyophilized secretome are determined before the secretome is lyophilized. In another preferred embodiment all the concentrations described for the components of the lyophilized adipose tissue mesenchymal stems cell secretome are determined after the lyophilized secretome is reconstituted.

In a preferred embodiment the lyophilized adipose tissue mesenchymal stems cell secretome is reconstituted in a solution comprising between 1 and 4 mL of diluent, preferably 1, 2, 3, or 4 mL of diluent, more preferably 2 mL of diluent.

The term "diluent", as used herein in reference to the reconstitution of the lyophilized formulation according to the invention, refers to a pharmaceutically acceptable solution which can be used to resuspend the lyophilized formulation without modifying the contents of the lyophilized formulation. Examples of such diluents are pharmaceutically acceptable buffers such as phosphate buffered saline buffer (PBS).

In another preferred embodiment the lyophilized formulation comprising adipose tissue mesenchymal stem cell secretome was obtained by a method devoid of ultracentrifugation. The term "ultracentrifugation" refers to high speed centrifugation which makes use of high centrifugal force for studying properties of biological particles at exceptionally high speeds. Current ultracentrifuges can spin to as much as 150,000 rpm (equivalent to 1,000,000 x g). They are used to harvest all membrane vesicles derived from the plasma membrane, endoplasmic reticulum (ER) and Golgi membrane, endosomes, ribosomes, ribosomal subunits, plasmids, DNA, RNA and proteins in fixed-angle rotors. Compared to microcentrifuges or high-speed centrifuges, ultracentrifuges can isolate much smaller particles and, additionally, whilst microcentrifuges and supercentrifuges separate particles in batches (limited volumes of samples must be handled manually in test tubes or bottles), ultracentrifuges can separate molecules in batch or continuous flow systems.

In a preferred embodiment, the lyophilized formulation of the invention was obtained by an *in vitro* method comprising the steps of:
i) providing adipose tissue comprising adipose tissue mesenchymal stromal cells;
ii) washing the adipose tissue of step i) obtaining a blood free lipid phase;
iii) separating the oil layer and the fat layer and discarding the oil layer obtaining a fat rich solution;
iv) digesting the collagen of the fat rich solution and discarding the oil layer obtaining a suspension;
v) washing and filtering the suspension to obtain a cell suspension;
vi) culturing the cell suspension to obtain a secretome;
vii) concentrating the secretome to obtain an exosome-rich secretome;
viii) adding trehalose to the exosome rich-secretome obtained in (vii); and
ix) lyophilizing the exosome rich-secretome obtained in (viii).

In a preferred embodiment step (ii) is carried out by adding physiological saline to the adipose tissue in a 1:1 ratio (v/v), agitating the mix leading to the separation of the aqueous and lipid phases of the adipose tissue and discarding the aqueous phase.

The term "physiological saline", as used herein, refers to a mixture of sodium chloride and water at a physiological pH and physiological osmolarity. In a preferred embodiment the physiological saline is a solution of 0.90% w/v of NaCl, 308 mOsm/L or 9.0 g per liter.

In a preferred embodiment step (iii) is carried out by adding a phosphate buffer to the lipid phase obtained in step (ii) in a 1:1 ratio (v/v) and centrifuging at between 350G to 550G for 3min to 6 min followed by removing the oil layer. Preferably, centrifuging at 450G for 5 minutes.

A phosphate buffer solution, commonly known as PBS, refers to a water-based salt solution containing disodium hydrogen phosphate, sodium chloride and, in some formulations, potassium chloride and potassium dihydrogen phosphate. The buffer helps to maintain a constant pH.

The rate of centrifugation can be specified by the angular velocity usually expressed as revolutions per minute (RPM), or acceleration expressed as G or g. The conversion factor between RPM and g depends on the radius of the centrifuge rotor. The particles' settling velocity in centrifugation is a function of their size and shape, centrifugal acceleration, the volume fraction of solids present, the density difference between the particle and the liquid, and the viscosity. The general formula for calculating the revolutions per minute (RPM) of a centrifuge is $RPM = \sqrt{\frac{g}{r}}$*,* where g represents the relative centrifugal force (RCF) and r the radius from the center of the rotor to a point in the sample.

The "oil layer" refers to the layer which sits on top of the suspension and derives from disrupted adipocytes.

In a preferred embodiment, step iv) is carried out by adding a collagenase solution to the fat rich solution at a 1:1 ratio (collagenase:fat - v/v).

The term "collagenase", as used herein, refers to an enzyme that breaks the peptide bonds in collagen. Preferably, the collagenase solution is a solution of collagenase in Hank's balanced salt solution.

In a preferred embodiment, the digestion is carried out for between 2h to 4 h, at 37°C and 5% CO₂, with agitation every 15-30 min. In a preferred embodiment the digestion is carried out for 3 h.

In another preferred embodiment, the digestion is stopped by the addition of fetal bovine serum.

In a preferred embodiment, the washing of step (v) is carried out by adding a phosphate buffer to the suspension obtained in step (iv) in a 1:1 ratio (v/v) and centrifuging at between 350G to 550G for 3 min to 6 min followed by removing the supernatant. Preferably at 450G for 5 minutes.

In a more preferred embodiment, after the previous step phosphate buffer was added to the pellet in a 1:4 ratio of pellet phosphate buffer (w/v), resuspending the pellet and centrifuging at between 350G to 550G for 3 to 15 minutes; more preferably centrifuging at 450G for 10 minutes or at 450G for 5 minutes.

In another preferred embodiment, the filtering of step (v) is carried out by passing the resuspended pellet by a 70 micrometer (µm) pore diameter filter.

In another preferred embodiment, after filtering the suspension was centrifuged at between 350G to 550G for 3 to 6 minutes and the supernatant was discarded to obtain a cell suspension. Preferably was centrifuged at 450G for 5 minutes.

In another preferred embodiment, step (vi) is carried out by culturing the cell suspension in DMEM/F12-C medium at a ratio 1:9 cell suspension: medium (v/v) at 37°C and 5% CO₂ for between 60 to 84 hours, preferably 72 hours, to obtain a secretome. Preferably, the medium is supplemented with 10% FBS, 1% L-glutamine, and 1% penicillin/streptomycin. Optionally, this medium has 1g/L glucose. In a preferred embodiment, the cell suspension contains 15 million cells.

In another preferred embodiment, step (vii) is carried out by centrifuging the secretome first at 5000 g for 20 minutes and subsequently centrifuging the supernatant at 4000 g for 1 hour to obtain an exosome-rich secretome. In a further preferred embodiment, the step (ix) is carried out by freezing the exosome-rich secretome for 1h at -80°C, following by 1 h at -60°C followed by a first drying step carried out at -60°C for 4h at a pressure of 120 microMercury (uHg), followed by a second drying step at -20°C for 8h at a pressure of 120 uHg.

In another preferred embodiment, the trehalose is added at a concentration of 25 mM.

In a preferred embodiment, the steps ii) to v) of the method according to the invention can be repeated one or more times. The skilled person in the art is well aware of the conditions required for ensuring the obtaining of a proper lyophilized secretome and if it requires that one or more of the steps mentioned are repeated.

An additional aspect of the invention is a lyophilized formulation comprising:
a) an adipose tissue MSC secretome comprising extracellular vesicle particles, secretome proteins; and
b) trehalose,
   preferably said lyophilized formulation is obtainable by an *in vitro* method comprising the steps of:
   i) providing adipose tissue comprising adipose tissue mesenchymal stromal cells;
   ii) washing the adipose tissue of step i) obtaining a blood free lipid phase;
   iii) separating the oil layer and the fat layer and discarding the oil layer obtaining a fat rich solution;
   iv) digesting the collagen of the fat rich solution and discarding the oil layer obtaining a suspension;
   v) washing and filtering the suspension to obtain a cell suspension;
   vi) culturing the cell suspension to obtain a secretome;
   vii) concentrating the secretome to obtain an exosome-rich secretome;
   viii) adding trehalose to the exosome rich-secretome obtained in (vii); and
   ix) lyophilizing the exosome rich-secretome obtained in (viii).

All the aspects and embodiments disclosed herein are also applicable to the above lyophilized formulation. Preferably, the lyophilized formulation disclosed above has been obtained by a method that does not comprise a step of ultracentrifugation. In a preferred embodiment the secretome proteins of component (a) are not proteins forming part of vesicles, preferably are proteins forming part of the soluble fraction of the secretome.

Another aspect of the present invention relates to a pharmaceutical composition comprising the lyophilized formulation according to the first aspect of the invention and a pharmaceutically acceptable diluent.

The person skilled in the art will acknowledge that the secretome lyophilized or reconstituted according to the invention is suitable for use in medicine and may be presented accompanied by a pharmaceutically acceptable carrier. As used herein, the term "pharmaceutically acceptable carrier" means a non- toxic, inert solid, semi-solid or liquid filler, diluent, encapsulating material or formulation auxiliary of any type. Remington's Pharmaceutical Sciences. Ed. by Gennaro, Mack Publishing, Easton, Pa., 1995 discloses various carriers used in formulating pharmaceutical compositions and known techniques for the preparation thereof. Preferably, the carrier is a diluent.

Accordingly, the compositions comprising the lyophilized formulation according to the first aspect of the invention and a pharmaceutically acceptable diluent are pharmaceutical compositions.

In an embodiment, the diluent is selected from the group consisting of water for injection, physiological saline, phosphate buffered saline, purified water, and deionized water. Preferably is phosphate buffered saline.

In another embodiment, the pharmaceutical composition is a suspension.

In another embodiment, the pharmaceutical composition is an injectable formulation.

In another embodiment, the pharmaceutical composition is a reconstituted lyophilized formulation with 2 ml of diluent.

In another embodiment, the pharmaceutical composition has an average particle size of 167.6 ± 9.2 nm. Preferably, the particle size is determined by light scattering and Brownian motion analysis.

In an embodiment, the pharmaceutical composition of the invention contains a concentration of particles of between 1x10⁷ particles/ml to about 1x10¹¹ particles /ml, preferably 3.70 × 10⁹ ± 2.31 × 10⁸ particles/ml.

In a preferred embodiment the concentration of any one protein in the pharmaceutical composition after reconstitution of the lyophilized formulation according to the invention is between 1 ng/mL to between 100 µg/mL, between 1 ng/mL to 1000 ng/mL, between 50 ng/mL to 950 ng/mL, between 100 ng/mL to 900 ng/mL, between 150 ng/mL to 850 ng/mL, between 200 ng/mL to 800 ng/mL, between 250 ng/mL to 750 ng/mL, between 300 ng/mL to 700 ng/mL, between 350 ng/mL to 650 ng/mL, between 400 ng/mL to 600 ng/mL, between 450 ng/mL to 550 ng/mL, between 500 ng/mL to 550 ng/mL, between 1 µg/mL to 100 µg/mL, between 5 µg/mL to 95 µg/mL, between 10 µg/mL to 90 µg/mL, between 15 µg/mL to 85 µg/mL, between 20 µg/mL to 80 µg/mL, between 25 µg/mL to 75 µg/mL, between 30 µg/mL to 70 µg/mL, between 35 µg/mL to 65 µg/mL, between 40 µg/mL to 60 µg/mL, between 45 µg/mL to 55 µg/mL, or between 50 µg/mL to 55 µg/mL, preferably wherein the reconstitution is performed with 2 mL to 10 mL of a diluent. In another preferred embodiment the concentration of all of the proteins in the pharmaceutical composition after reconstitution of the lyophilized formulation according to the invention is of between 1 µg/mL to between 100 µg/mL, between 1 ng/mL to 1000 ng/mL, between 50 ng/mL to 950 ng/mL, between 100 ng/mL to 900 ng/mL, between 150 ng/mL to 850 ng/mL, between 200 ng/mL to 800 ng/mL, between 250 ng/mL to 750 ng/mL, between 300 ng/mL to 700 ng/mL, between 350 ng/mL to 650 ng/mL, between 400 ng/mL to 600 ng/mL, between 450 ng/mL to 550 ng/mL, between 500 ng/mL to 550 ng/mL, between 1 µg/mL to 100 µg/mL, between 5 µg/mL to 95 µg/mL, between 10 µg/mL to 90 µg/mL, between 15 µg/mL to 85 µg/mL, between 20 µg/mL to 80 µg/mL, between 25 µg/mL to 75 µg/mL, between 30 µg/mL to 70 µg/mL, between 35 µg/mL to 65 µg/mL, between 40 µg/mL to 60 µg/mL, between 45 µg/mL to 55 µg/mL, or between 50 µg/mL to 55 µg/mL, preferably wherein the reconstitution is performed with 2 mL to 10 mL of a diluent, preferably 2 mL diluent.

In a preferred embodiment the concentration of growth factors in the pharmaceutical composition after reconstitution of the lyophilized formulation according to the invention is between 1 ng/mL to between 100 µg/mL, between 1 ng/mL to 1000 ng/mL, between 50 ng/mL to 950 ng/mL, between 100 ng/mL to 900 ng/mL, between 150 ng/mL to 850 ng/mL, between 200 ng/mL to 800 ng/mL, between 250 ng/mL to 750 ng/mL, between 300 ng/mL to 700 ng/mL, between 350 ng/mL to 650 ng/mL, between 400 ng/mL to 600 ng/mL, between 450 ng/mL to 550 ng/mL, between 500 ng/mL to 550 ng/mL, between 1 µg/mL to 100 µg/mL, between 5 µg/mL to 95 µg/mL, between 10 µg/mL to 90 µg/mL, between 15 µg/mL to 85 µg/mL, between 20 µg/mL to 80 µg/mL, between 25 µg/mL to 75 µg/mL, between 30 µg/mL to 70 µg/mL, between 35 µg/mL to 65 µg/mL, between 40 µg/mL to 60 µg/mL, between 45 µg/mL to 55 µg/mL, or between 50 µg/mL to 55 µg/mL, preferably wherein the reconstitution is performed with 2 mL to 10 mL of a diluent, preferably 2 mL diluent.

In another preferred embodiment the concentration of cytokines in the pharmaceutical composition after reconstitution of the lyophilized formulation according to the invention is between 1 ng/mL to between 100 µg/mL, between 1 ng/mL to 1000 ng/mL, between 50 ng/mL to 950 ng/mL, between 100 ng/mL to 900 ng/mL, between 150 ng/mL to 850 ng/mL, between 200 ng/mL to 800 ng/mL, between 250 ng/mL to 750 ng/mL, between 300 ng/mL to 700 ng/mL, between 350 ng/mL to 650 ng/mL, between 400 ng/mL to 600 ng/mL, between 450 ng/mL to 550 ng/mL, between 500 ng/mL to 550 ng/mL, between 1 µg/mL to 100 µg/mL, between 5 µg/mL to 95 µg/mL, between 10 µg/mL to 90 µg/mL, between 15 µg/mL to 85 µg/mL, between 20 µg/mL to 80 µg/mL, between 25 µg/mL to 75 µg/mL, between 30 µg/mL to 70 µg/mL, between 35 µg/mL to 65 µg/mL, between 40 µg/mL to 60 µg/mL, between 45 µg/mL to 55 µg/mL, or between 50 µg/mL to 55 µg/mL, preferably wherein the reconstitution is performed with 2 mL to 10 mL of a diluent, preferably 2 mL diluent.

The pharmaceutical compositions of this invention, as well as the lyophilized formulations according to the invention, can be administered to a patient by any means known in the art including oral and parenteral routes. According to such embodiments, inventive compositions may be administered by injection (e.g., intravenous, subcutaneous or intramuscular, intraperitoneal injection), rectally, vaginally, topically (as by powders, creams, ointments, or drops), or by inhalation (as by sprays). Preferably, they are administered by injection.

Another aspect of the present invention relates to the lyophilized formulation according to the first aspect of the invention or to the pharmaceutical composition according to the second aspect of the invention for use in medicine. Preferably in regenerative medicine.

Another aspect of the present invention relates to the lyophilized formulation according to the first aspect of the invention or to the pharmaceutical composition according to the second aspect of the invention for use in the prevention and/or treatment of joint lesions. Preferably, the joint lesion is an articular cartilage defect or a subchondral bone defect.

A further aspect of the present invention relates to a method of prevention and/or treatment of a patient with joint lesions, the method comprising the administration to the patient of the lyophilized formulation according to the first aspect of the invention or of the pharmaceutical composition according to the invention. Preferably, the joint lesion is an articular cartilage defect or a subchondral bone defect.

Another aspect of the present invention relates to the use of the lyophilized formulation according to the first aspect of the invention or of the pharmaceutical composition according to the invention for the manufacture of a medicament for the prevention and/or treatment of join lesions. Preferably, the joint lesion is an articular cartilage defect or a subchondral bone defect.

A further aspect of the present invention relates to the lyophilized formulation according to the first aspect of the invention or to the pharmaceutical composition according to the second aspect of the invention for use in the prevention and/or treatment of a degenerative joint disease, preferably osteoarthritis.

A further aspect of the present invention relates to a method of prevention and/or treatment of a patient with degenerative joint disease, preferably osteoarthritis, the method comprising the administration to the patient of the lyophilized formulation according to the first aspect of the invention or of the pharmaceutical composition according to the invention.

Another aspect of the present invention relates to the use of the lyophilized formulation according to the first aspect of the invention or of the pharmaceutical composition according to the invention for the manufacture of a medicament for the prevention and/or treatment of degenerative joint disease, preferably osteoarthritis.

All embodiments in relation to the lyophilized formulation according to the first aspect of the invention or of the pharmaceutical composition according to the invention for use in treatments are equally valid for the aspects relating to a method for treatment and for the use in the manufacture of a medicament.

The term "prevention", as used herein, relates to the capacity to prevent, minimize, or hinder the onset or development of a disease or condition before its onset. When referred to joint lesions, the term "prevention" also encompasses the administration of the lyophilized formulation according to the first aspect of the invention or to the pharmaceutical composition prior to the onset of joint lesions or of a degenerative joint disease, preferably osteoarthritis as well as the administration of the lyophilized formulation according to the first aspect of the invention or to the pharmaceutical composition after the onset of articular cartilage defect but before the onset of subchondral bone defect, or vice-versa.

As used herein, the terms "treat", "treatment" and "treating" refer to the reduction or amelioration of the progression, severity and/or duration of a condition, disorder or disease, or the amelioration of one or more symptoms (preferably, one or more discernible symptoms) of a condition, disorder or disease. The terms "treat", "treatment" and "treating" also refer to the amelioration of at least one measurable physical parameter of a condition, disorder or disease not necessarily discernible by the patient. Furthermore, "treat", "treatment" and "treating" refer also to the inhibition of the progression of a condition, disorder or disease, either physically by, e.g., stabilization of a discernible symptom, physiologically by, e.g., stabilization of a physical parameter, or both. "Treat", "treatment" and "treating" may refer, too, to the reduction or stabilization of the condition, disorder or disease.

It will be understood by the person skilled in the art that by use in medicine, the lyophilized formulation or pharmaceutical composition according to the invention can be administered to a patient in order to induce a therapeutic response.

The therapeutic response comprises the suppression, reduction or arrest of the causes of the pathological condition or the disease suffered by a patient; the elimination, reduction, arrest or amelioration of the symptoms of the condition or disease; or the extinction, arrest or slowing down of the progression of the condition or disease in the patient.

The term "osteoarthritis" refers to a disease caused by damage to the articular cartilage and the tibial tissue underneath it. Rheumatoid arthritis is one type of arthritis, but osteoarthritis is the most common form of arthritis. Symptoms include joint pain and stiffness, and pain is felt only when moving at first, but when it becomes chronic, pain is felt continuously. It occurs more frequently with age, and millions of people around the world suffer from this disease. It is a representative degenerative disease that continues to progress once it occurs. Common initial symptoms include difficulty moving the joint and pain. All joints can show symptoms, but symptoms are mainly seen in the fingers, neck, lower spine, knees, and sacrum.

In a preferred embodiment the treatment of articular cartilage is a regenerative treatment.

The term "regenerative treatment", as used herein, refers to the treatment to recover, regenerate or restore articular cartilage which has been damaged due to disease. The term "cartilage," "articular cartilage," and "cartilage tissue", as used herein, is generally recognized in the art, and refers to the hyaline cartilage that covers the surface of articulating joints. Hyaline cartilage, for example, comprises chondrocytes surrounded by a dense extra-cellular matrix consisting of type II collagen, proteoglycans and water. Various other forms of cartilage exist throughout the body; for example, fibrocartilage, elastic cartilage, and hyaline cartilage that exists in non-articulating or minimally-articulating interfaces of the body.

As used herein, "cartilage defect" refers to cartilage tissue that is missing, reduced in quantity or otherwise damaged. A cartilage tissue defect may result from a congenital anomaly, disease, disease treatment, or trauma, and may be acute or chronic (osteoarthritis).

In another embodiment, the lyophilized formulation or pharmaceutical composition of the invention is for use in the treatment of subchondral bone defect.

The term "subchondral bone", as used herein, refers to the bony layer beneath the hyaline cartilage and cement line and can be divided into two parts anatomically, the subchondral bone plate (SBP) and subchondral bone trabecula. The SBP is a compact, polyporous calcified plate crisscrossed by multiple vessels and nerve fibers. In the context of the present application, including in the claims, the term "subchondral bone defect" should be interpreted as including any subchondral bone defect, including but not limited to a fracture, a lesion, edema, a tumor, and/or sclerotic hardening, by way of example.

As used herein, "bone defect" refers to bone that is broken, fractured, missing portions or otherwise damaged. Such damage may be due to congenital anomaly, disease, disease treatment, trauma or osseous infection, and may be acute or chronic. For instance, bone loss may occur as a result of tumor resection, thus resulting in a bone defect. Non-limiting examples of bone defects include: bone fractures, bone/spinal deformation, bone dysplasia, scoliosis, osteroporosis, osteomalacia, rickets, fibrous osteitis, fibrous dysplasia, renal bone dystrophy, and Paget's disease of bone.

In a preferred embodiment, the efficacy of the lyophilized formulation or pharmaceutical composition of the invention in the treatment of joint lesions is evaluated by one or more of the following parameters: cartilage edge regeneration, chondrocyte presence in transitional zones, extracellular matrix degeneration (proteoglycans), subchondral bone vascularization, trabecular bone regeneration, type II collagen formation, and cartilage fissures.

The evaluation parameters as used herein refer to:
- **Cartilage edge regeneration:** Assessment of healing at the interface between healthy and damaged cartilage.
- **Chondrocyte presence in transitional zones:** Evaluation of chondrocyte repopulation, essential for cartilage maintenance.
- **Extracellular matrix regeneration (proteoglycans):** Quantifying proteoglycan deposition in the regenerated matrix.
- **Subchondral bone vascularisation:** Observation of new blood vessel formation, crucial for bone repair.
- **Trabecular bone regeneration:** Reconstruction of subchondral trabecular structures.
- Type II collagen formation: The presence and organisation of type II collagen are hallmarks of hyaline cartilage regeneration.
- Cartilage fissures: Analysis of the closure and repair of fissures in the regenerated cartilage.

The evaluation parameters are known to the skilled person in the art, and can be evaluated as described in the Example section of this description specification.

As previously mentioned, the lyophilized formulation or pharmaceutical composition of the invention can be administered to the patient in several ways. In a preferred embodiment, the administration is done by intra-lesion injection. In another embodiment, the administration is done around the lesion or near the lesion. In a more preferred embodiment, the administration is done by intra-articular injection, preferably at the lesion site. In a more preferred embodiment, it is injected at the suprapatellar bursa. In a preferred embodiment, the administration is guided by ultrasound.

In another preferred embodiment the administration is done with between 1 to 10 mL, preferably 1 mL, 2 mL, 3 mL, 4 mL, 5 mL, 6 mL, 7 mL, 8 mL, 9 mL, 10 mL, more preferably 2 mL. Even more preferably is done with a 2 mL reconstituted lyophilized formulation.

In a preferred embodiment, the administration is done one, two, three, four or more times. In a preferred embodiment, the administration is done two or more times, wherein the second administration is done between 2 and 14 days after the first one, preferably 7 days after the first one, and the third and fourth administrations are done at between 7 and 21 days intervals from the previous administration, preferably 15 days from the previous administration.

As the skilled person will be aware, the adipose tissue mesenchymal stem cell secretome being administered can be derived from the subject/patient being administered it, or from another subject. Hence, in a preferred embodiment the adipose tissue mesenchymal stems cell secretome is allogeneic to the subject/patient being treated.

The term "allogeneic" generally refers to cells of the same species that are genetically different from the cells being compared.

In another embodiment, the adipose tissue mesenchymal stems cell secretome is autologous to the subject being treated.

The term "autologous" refers to the fact that the adipose tissue mesenchymal stems cell secretome used to treat a subject is obtained from adipose tissue obtained from the same subject being treated.

In a preferred embodiment, the subject/patient to be treated is a mammal of any age, race or gender.. In another preferred embodiment, the mammal is selected from a group consisting of: non-human primates, such as chimpanzees and other apes and monkey species, farm animals or livestock, such as cattle, sheep, lambs, pigs, goats and horses; domestic mammals, such as dogs and cats, laboratory animals, such as mice, rats and guinea pigs, aardvarks, antelopes, armadillos, badgers, bats, bears, bobcats, buffalo, camels, large cats, cheetahs, cougars, coyotes, deer, dolphins, donkeys, elephants, elk, ermine, ferrets, foxes, giraffes, goats, guanacos, hedgehogs, hippopotamuses, hyenas, jaguars, leopards, lions, llamas, lynxes, manatees, marsupials, mink, moles, moose, mules, mustelids, ocelots, pine marten, pinnipeds, rabbits, raccoons, pandas, reindeer, caribou, rhinoceroses, tigers, vicunas, weasels, whales, wolverine, wolves, yaks, and zebras. In another preferred embodiment, the mammal is a human.

A final aspect of the present invention refers to a kit comprising the lyophilized formulation according to the first aspect of the invention and a pharmaceutically acceptable diluent.

In a preferred embodiment, the pharmaceutically acceptable diluent is selected from the group consisting of water for injection, physiological saline, phosphate buffered saline, purified water, and deionized water.

The term "kit", in the context of the current invention, is understood as a product of the different reagents for performing the methods and/or treatments described in the present invention, in which the kit comprises the lyophilized formulation comprising adipose tissue mesenchymal stems cell secretome of the invention and a reconstituting solution. Suitable materials for packaging the components of the kit include, without being limited to, glass, plastic (polyethylene, polypropylene, polycarbonate and the like), bottles, vials, paper, sachets and the like. Kits can additionally contain instructions for using the different components in the kit. Said instructions can be in printed format or in an electronic device capable of storing instructions such that they can be read by a person, such as electronic storage media (magnetic discs, tapes and the like), optical means (CD-ROM, DVD, USB) and the like. The media can additionally or alternatively contain Internet addresses where said instructions are provided.

The present invention is further defined by the following aspects:
1. A lyophilized formulation comprising:
   a) an adipose tissue mesenchymal stems cell (MSC) secretome, said secretome comprising:
      (i) extracellular vesicle particles; and
      (ii) at least one protein selected from a group consisting of: extracellular matrix protein 2 (ECM2), Prolargin, Vitamin D-binding protein (DBP), Midkine and any combination thereof; preferably a combination of extracellular matrix protein 2 (ECM2), Prolargin Vitamin D-binding protein (DBP) and Midkine;
         and
   b) a cryoprotectant, wherein said cryoprotectant is trehalose.
2. The lyophilized formulation according to aspect 1, wherein the extracellular vesicle particles are selected from the group consisting of exosomes, microvesicles, apoptotic bodies and combinations thereof.
3. The lyophilized formulation according to aspect 2, wherein the extracellular vesicle particles are exosomes.
4. The lyophilized formulation according to any one of aspects 1 to 3, wherein the secretome further comprises one or more of the proteins selected from the group consisting of: Secreted frizzled-related protein 4 (SFRP4), Tissue factor pathway inhibitor (TFPI), Adipocyte enhancer-binding protein 1 (AEBP1), Dickkopf-related protein 3 (DKK3), Plasminogen, and Endoglin.
5. The lyophilized formulation according to any one of aspects 1 to 4, wherein the concentration of trehalose before lyophilization is between 20 mM to between 30 mM.
6. The lyophilized formulation according to aspect 5, wherein the concentration of trehalose is of 25 mM before lyophilization.
7. The lyophilized formulation according to any one of aspects 1 to 6, wherein the adipose tissue MSC secretome is a human adipose tissue MSC secretome.
8. The lyophilized formulation according to any one of aspects 1 to 7, obtained by an *in vitro* method comprising the steps of:
   i) providing adipose tissue comprising adipose tissue mesenchymal stromal cells;
   ii) washing the adipose tissue of step i) obtaining a blood free lipid phase;
   iii) separating the oil layer and the fat layer and discarding the oil layer obtaining a fat rich solution;
   iv) digesting the collagen of the fat rich solution and discarding the oil layer obtaining a suspension;
   v) washing and filtering the suspension to obtain a cell suspension;
   vi) culturing the cell suspension to obtain a secretome;
   vii) concentrating the secretome to obtain an exosome-rich secretome;
   viii) adding trehalose to the exosome rich-secretome obtained in (vii); and
   ix) lyophilizing the exosome rich-secretome obtained in (viii).
9. The lyophilized formulation according to aspect 8, wherein the *in vitro* method does not comprise a step of ultracentrifugation.
10. The lyophilized formulation according to any one of aspects 8 or 9, wherein step (ii) is carried out by adding physiological saline to the adipose tissue in a 1:1 ratio, agitating the mix leading to the separation of the aqueous and lipid phases of the adipose tissue and discarding the aqueous phase.
11. The lyophilized formulation according to any one of aspects 8 to 10, wherein step (iii) is carried out by adding a phosphate buffer to the lipid phase obtained in step (ii) in a 1:1 ratio (v/v) and centrifuging at between 350G to 550G for 3 min to 6 min followed by removing the oil layer.
12. The lyophilized formulation according to any one of aspects 8 to 11, wherein step (iv) is carried out by adding a collagenase solution to the fat rich solution at a 1:1 ratio (collagenase:fat - v/v).
13. The lyophilized formulation according to aspect 12, wherein the collagenase solution is a solution of collagenase in Hank's balanced salt solution.
14. The lyophilized formulation according to any one of aspects 8 to 13, wherein the digestion is carried out for between 2 h to 4 h, at 37°C and 5% CO₂, with agitation every 15-30 min.
15. The lyophilized formulation according to any one of aspects 8 to 14, wherein the digestion is stopped by the addition of fetal bovine serum.
16. The lyophilized formulation according to any one of aspects 8 to 15, wherein the washing of step (v) is carried out by adding a phosphate buffer to the suspension obtained in step (iv) in a 1:1 ratio (v/v) and centrifuging at between 350G to 550G for 3 min to 6 min followed by removing the supernatant.
17. The lyophilized formulation according to aspect 16, wherein phosphate buffer was added to the pellet in a 1:4 ratio of pellet phosphate buffer (w/v), resuspending the pellet and centrifuging at between 350 G to 550 G for 3 to 15 minutes.
18. The lyophilized formulation according to any one of aspects 8 to 17, wherein the filtering of step (v) is carried out by passing the resuspended pellet by a 70 µm pore diameter filter.
19. The lyophilized formulation according to aspect 18, wherein the suspension was centrifuged at between 350 G to 550 G for 3 to 6 minutes and the supernatant was discarded to obtain a cell suspension.
20. The lyophilized formulation according to any one of aspects 8 to 19, wherein step (vi) is carried out by culturing the cell suspension in DMEM/F12-C medium at a ratio 1:9 cell suspension: medium (v/v) at 37°C and 5% CO₂ for 72 hours, the medium preferably supplemented with 10% FBS, 1% L-glutamine, and 1% penicillin/streptomycin to obtain a secretome.
21. The lyophilized formulation according to any one of aspects 8 to 20, wherein step (vii) is carried out by centrifuging the secretome first at 5000 g for 20 minutes and subsequently centrifuging the supernatant at 4000 g for 1 hour to obtain an exosome-rich secretome.
22. The lyophilized formulation according to any one of aspects 8 to 21, wherein step (ix) is carried out by freezing the exosome-rich secretome for 1h at -80°C, following by 1h at -60°C followed by a first drying step carried out at -60°C for 4h at a pressure of 120 uHg, followed by a second drying step at -20°C for 8h at a pressure of 120 uHg.
23. The lyophilized formulation according to any one of aspects 8 to 22, wherein the trehalose is added at a concentration of 25 mM.
24. A pharmaceutical composition comprising the lyophilized formulation according to any one of aspects 1 to 23 and a pharmaceutically acceptable diluent.
25. The pharmaceutical composition according to aspect 24, wherein the diluent is selected from the group consisting of water for injection, physiological saline, phosphate buffered saline, purified water, and deionized water.
26. The pharmaceutical composition according to any one of aspects 24 or 25, wherein the pharmaceutical composition is a suspension.
27. The pharmaceutical composition according to any one of aspects 24 to 26, wherein the pharmaceutical composition is an injectable formulation.
28. The pharmaceutical composition according to any one of aspects 24 to 27, wherein the said composition is a reconstituted lyophilized formulation with 2 ml of diluent.
29. The pharmaceutical composition according to any one of aspects 24 to 28, wherein the average particle size is of 167.6 ± 9.2 nm.
30. The pharmaceutical composition according to aspect 29, wherein the particle size is determined by light scattering and Brownian motion analysis.
31. The pharmaceutical composition according to any one of aspects 24 to 30, wherein the concentration of particles is of 3.70 × 10⁹ ± 2.31 × 10⁸ particles/ml.
32. The pharmaceutical composition according to any one of aspects 24 to 31, wherein the concentration of any one of the proteins after reconstitution of the lyophilized formulation is between 1 ng/mL to between 100 µg/mL.
33. The lyophilized formulation according to any one of aspects 1 to 23 or the pharmaceutical composition according to any one of aspects 24 to 32 for use in medicine, preferably in regenerative medicine.
34. The lyophilized formulation according to any one of aspects 1 to 23 or the pharmaceutical composition according to any one of aspects 24 to 32 for use in the prevention and/or treatment of a joint lesion.
35. The lyophilized formulation or pharmaceutical composition for use according to aspect 34, wherein the joint lesion is an articular cartilage defect or a subchondral bone defect.
36. The lyophilized formulation according to any one of aspects 1 to 23 or the pharmaceutical composition according to any one of aspects 24 to 32 for use in the prevention and/or treatment of a degenerative joint disease.
37. The lyophilized formulation or the pharmaceutical composition for use according to aspect 36, wherein the degenerative joint disease is osteoarthritis.
38. The lyophilized formulation or pharmaceutical composition for use according to any one of aspects 33 to 37, wherein the administration is done by intra-articular injection, preferably at the lesion site, more preferably at the suprapatellar bursa.
39. The lyophilized formulation or pharmaceutical composition for use according to any one of aspects 33 to 38, wherein the administration is done with a 2 ml reconstituted lyophilized formulation.
40. The lyophilized formulation or pharmaceutical composition for use according to any one of aspects 33 to 39, wherein the administration is done one, two, three, four or more times.
41. The lyophilized formulation or pharmaceutical composition for use according to aspect 40, wherein the second administration is done 7 days after the first one, and the third and fourth administrations are done at 15 days interval from the previous administration.
42. The lyophilized formulation or pharmaceutical composition for use according to any one of aspects 33 to 41, wherein the adipose tissue MSC secretome is allogeneic to the subject being treated.
43. The lyophilized formulation or pharmaceutical composition for use according to any one of aspects 33 to 42, wherein the subject is a human.
44. A kit comprising the lyophilized formulation according to any one of aspects 1 to 23 and a pharmaceutically acceptable diluent.
45. The kit according to aspect 44, wherein the pharmaceutically acceptable diluent is selected from the group consisting of water for injection, physiological saline, phosphate buffered saline, purified water, and deionized water.

The following examples are provided as merely illustrative and are not to be construed as limiting the scope of the invention.

### EXAMPLES

### Example 1

### Materials and methods

### Sample Description

This experimental study involved 36 lambs selected for their joint characteristics, closely resembling human joints. Thus, they are suitable models for investigating articular regeneration.

Each lamb underwent surgically induced articular lesions to evaluate the regenerative capacity of the applied treatments (Figure 1). The procedure was performed under spinal anaesthesia using 2% Mepivacaine, with a volumetric range of 2 to 3 cc per animal. Anesthesia was achieved within a maximum of 1 minute, with an anaesthetic effect lasting between 55 and 90 minutes. The lumbosacral area was shaved, washed with antiseptic soap, and disinfected with 2% chlorhexidine for skin antisepsis prior to the spinal technique. The average surgical time per animal was 15 minutes. For the surgical preparation of the femorotibial-patellar joint, the area was shaved, washed with antiseptic soap, and disinfected with 2% chlorhexidine for skin asepsis. The lesions were created using a trocar to remove cartilage and subchondral bone, replicating conditions suitable for assessing therapeutic interventions in joint degeneration.

### Experimental Groups

The 36 lambs were randomly divided into four experimental groups to ensure homogeneity across individuals. Unfortunately, one of the lambs died due to external circumstances related to a fight with another male.

The Control Group received no regenerative treatment, serving as a baseline to assess the natural capacity for tissue regeneration. The Fresh Secretome Group was treated with hAD-MSC-derived secretome containing exosomes that were freshly prepared and administered immediately via intra-articular injection at the lesion site, maximising their regenerative potential. In the Cryopreserved Secretome Group, hAD-MSC-derived secretome containing exosomes were cryopreserved at ultra-low temperatures, thawed before use, and then injected intra-articularly, allowing the evaluation of how cryopreservation affects their therapeutic properties. Lastly, the Lyophilized Secretome Group used dehydrated secretome containing exosomes through lyophilisation and reconstituted before being injected. This approach assessed whether lyophilisation's storage and transport benefits impacted the exosomes' regenerative efficacy.

### Secretome Isolation and Preparation

### Obtaining MSCs from Adipose Tissue

Human adipose tissue-derived mesenchymal stromal cells were isolated from a healthy 35-year-old female donor. Written informed consent was previously obtained according to institutional guidelines. The donor was tested for Human Immunodeficiency Virus 1 (HIV-1), Hepatitis C virus (HCV), Hepatitis B virus (HBV) and Human Immunodeficiency Virus 2 (HIV-2), with negative results.

The following monoclonal antibodies (mouse anti-human) were used for flow cytometric immunophenotyping of hAD-MSC: CD90 FITC (Fluorescein isothiocyanate), negative control PE (phycoerythrin) (CD34-clone 581; CD11b-clone ICRF44; CD19-clone HIB19; CD45-clone HI30; HLA-DR-clone G46-6), CD44 APCH7 (APC-cyanine tandem dye), CD105 PerCP-Cy 5.5 (physio-erythrin-cyanine 5.5), CD73 APC (Allophycocyanin). All of them were purchased from BD Biosciences (San José, CA). FACSAria Ill cell sorter flow cytometer a (BD Biosciences) was used for hAD-MSC sample acquisition. Labelled cells were acquired immediately after the staining using a FACSAria Ill cell sorter flow cytometer equipped with the Diva software program (BD Biosciences). hAD-MSC was characterised using the conjugated monoclonal antibody combinations in hAD-MSC (5 × 10⁵ cells) was suspended in PBS and incubated with combinations of the monoclonal antibodies described above and also was acquired hAD-MSC unstained used as control.

The collected adipose tissue was transferred to sterile containers, filling them halfway. Physiological saline was added to fill the container, and the mixture was vigorously inverted to separate the aqueous and lipid phases. The aqueous phase was then discarded. This process was repeated until the aqueous phase was clear of blood. 35-40 ml of the lipid phase was then transferred to falcon tubes. If the initial sample was small, a single centrifugation wash was performed, and phosphate buffered saline (PBS) 1x room temperature (RT) was added at this point.

The mixture was centrifuged at 450G for 5 minutes. The oil layer was carefully removed without disturbing the underlying fat layer. 35-40 ml of the lipid phase (including the white halo of loose MSCs) was then transferred to a new 50 ml falcon tube, and PBS 1x RT was added to reach a total volume of 50 ml. This mixture was centrifuged again at 450G for 5 minutes, and the oil layer was discarded. Finally, the vascular fat was transferred to a new sterile container.

A 1x collagenase solution was prepared by adding 10 ml of 10x collagenase to 90 ml of Hanks balanced salt solution (HBSS) RT in a sterile container and homogenising the mixture. The 1x collagenase solution was added to the vascular fat in a 1:1 ratio (collagenase: fat - v/v). The container was tightly closed and vigorously inverted. It was then incubated for 3 hours at 37°C and 5% CO₂, with agitation every 15-30 minutes. This digestion process forms three distinct layers: An oil layer at the top, which was carefully removed and discarded; a lipid phase containing vascular fat, adipocytes, connective tissue, and MSCs; and an aqueous phase containing saline/PBS 1x, collagenase, erythrocytes, and precipitated MSCs. This phase also includes a connective tissue clot. Fetal bovine serum RT was added to inactivate the collagenase.

PBS 1x RT was added to the mixture, and the pellet was gently resuspended. The mixture was then centrifuged at 450G for 5 minutes. The supernatant (containing PBS, FBS, and collagenase) was carefully removed, leaving the pellet as dry as possible without losing cells. PBS 1x RT was added to achieve a 1:4 ratio of pellet: PBS (w/v), and the pellet was gently resuspended. The suspension was transferred to a new 15 ml Falcon tube, avoiding the connective tissue clot. The mixture was centrifuged at 450G for 10 minutes, and the supernatant was discarded. This washing process was repeated twice with 10 ml of PBS 1x RT and centrifugation at 450G for 5 minutes each to ensure a cell pellet free of FBS and collagenase.

For the filtering and final preparation for seeding, the pellet was resuspended in a small amount of PBS 1x at room temperature and drawn into a syringe. The suspension was then passed through a 70 µm filter into a new 15 ml Falcon tube and centrifuged at 450G for 5 minutes. Following centrifugation, the supernatant was discarded, and the pellet was resuspended in DMEM/F12-C medium supplemented with 10% FBS, 1% L-Glutamine, and 1% P/S (penicillin/streptomycin), using 1 ml of cell suspension per flask. The medium, maintained at room temperature, could have a glucose concentration of either 1 g/L.

Cells were counted for cell counting and seeding using a Neubauer chamber and Trypan Blue staining to calculate the volume of cell suspension needed to seed 15 million cells per flask. The appropriate volume of DMEM-C medium was added to each flask to achieve a final volume of 10 ml, with the medium dispensed into the centre of the flask and gently swirled to ensure the entire surface was covered. The calculated volume of the homogenised cell suspension was then gently added to the centre of the flask, and the flask was carefully rocked back and forth to distribute the cell suspension evenly, avoiding vigorous movements that could cause cells to accumulate at the edges. Finally, the flasks were incubated at 37°C and 5% CO₂ in an incubator.

### Exosome Concentration and Lyophilisation Process

The centrifuge was prepared at 4°C before starting the procedure, and the entire process was conducted at this temperature. After 72 hours, the medium (secretome) was extracted and transferred to a 50 ml Falcon tube from the culture in the flask. The tube was centrifuged at 5000 g for 20 minutes to remove debris and dead cells. The supernatant was collected, and 20 ml was transferred to a clean, pre-sterilized Vivaspin tube (3K mwco) to avoid contamination. This was centrifuged at 4000 g for 1 hour. The upper volume (approximately 10 ml) was collected and stored in a Falcon tube.

If the exosome concentrate needed to be thawed from -80°C, it was sprayed with 70% alcohol and placed in an incubator for rapid thawing. The sample was then vortexed for 5 minutes to separate any aggregates that may have formed during freezing. The Trehalose concentration used was 25 mM.

A freezing cycle was first performed to lyophilise the exosomes, which involved freezing the samples for 1 hour at -80°C, followed by 1 hour at -60°C. This was then followed by the primary drying phase, where the samples were dried for 4 hours at -60°C at a pressure of 120 uHg. After this, a secondary drying phase took place, with the samples being dried for 8 hours at -20°C at the same pressure of 120 uHg.

To ensure the integrity of the samples, sealing was performed under vacuum conditions or quickly under GMP conditions. The lyophilised exosomes were initially stored at -80°C and then at -20°C (the optimal temperature to maintain the stability of the molecules over time).

### Flow Cytometric Characterization of hAD-MSC Surface Marker Expression

Flow cytometric analysis was performed on passage three cells after 21 days of culture to assess the expression of surface antigens characteristic of hAD-MSCs. Cells were incubated with antibodies targeting specific surface markers, and their expression was evaluated by comparing the percentage of positive cells (light grey histograms) with the negative control (dark grey histograms), as shown in Figure 2.

Figure 2A illustrates the negative selection cocktail, which includes markers CD45, CD34, CD11b, CD19, and HLA-DR, conjugated with phycoerythrin (PE). These markers are typically absent in hAD-MSCs, confirming their lack of hematopoietic or endothelial lineage. These markers exclude non-MSC cells, ensuring the remaining cells are mesenchymal stem cells.

The subsequent histograms depict the expression of key MSC markers. The high percentages of cells expressing CD44, CD90, CD73, and CD105 confirm the presence of MSCs, as these markers are characteristic of mesenchymal stem cells (Figure 2 B, C, D, E).

### Nanosight Extracellular Vesicle Quantification

Extracellular vesicle (EV) quantification and characterisation were performed using a NanoSight NS 300 system (NanoSight Ltd., UK) equipped with an sCMOS camera. This technology combines light scattering and Brownian motion analysis to determine particle size distribution and concentration in liquid suspension. Before analysis, EV samples were concentrated and diluted in 1 ml of double-filtered PBS. Measurements were conducted at 24°C with a viscosity range of 0.9-0.965 cP, 25 frames per second, and a 60-second measurement time. The detection threshold was consistent across all samples, with results representing the mean sizes and standard deviations of at least three measurements.

The results in Figure 3 demonstrated EV particle size and concentration variability across the experimental groups. In the control group, the residual product from Vivaspin filtration exhibited an average particle size of 75.9 ± 1.4 nm with a concentration of 1.78 × 10⁸ ± 4.29 × 10⁶ particles/ml (Figure 3A). The fresh group displayed a larger particle size of 153.6 ± 12.5 nm and a higher concentration of 1.38 × 10⁹ ± 2.02 × 10⁸ particles/ml (Figure 3B). The cryopreserved group showed the largest particle size (181.1 ± 3.1 nm) and the highest concentration (6.60 × 10⁹ ± 1.06 × 10⁸ particles/ml) (Figure 3C). The lyophilised group revealed a slightly smaller particle size of 167.6 ± 9.2 nm but still maintained a high concentration of 3.70 × 10⁹ ± 2.31 × 10⁸ particles/ml (Figure 3D).

### Intra-Articular Treatment of Sheep Following Cartilage Extraction Surgery

Following the initial cartilage extraction surgery, all sheep received prophylactic treatment with Oxytetracycline (20 mg/kg every 96 hours, administered twice) and Phenylbutazone (8 mg/kg every 24 hours for three days). Their behaviours were monitored, and body temperatures were recorded. The surgical site was evaluated every three days on three separate occasions.

Treatment was initiated 20 days after the cartilage extraction surgery, with each administration consisting of a 2 ml injection of the designated product (fresh, cryopreserved, or lyophilised). Injections were conducted using 3 ml syringes with 22G needles, following aseptic techniques before each injection. The first injection was administered 20 days post-surgery, with the second given one week later. The final two treatments were administered at 15-day intervals. The first three administrations were guided by ultrasound, with the formulations injected into the suprapatellar bursa. A Mindray Z5-Vet ultrasound machine, utilising a microconvex transducer at a frequency of 6.5 MHz, facilitated these injections. The fourth treatment did not require ultrasound guidance, as the periarticular inflammation had diminished, allowing for direct palpation of the joint.

### Histological Techniques

To assess treatment efficacy, detailed histological analyses were conducted on biopsies collected at three euthanasia time points, reflecting different stages of regeneration. A double biopsy was performed using a biopsy punch, starting from the centre of the lesion and extending towards the intact cartilage. This technique ensured the acquisition of a representative sample for histopathological analysis. The collected biopsy samples were stored in 37% formalin until analysis.

Three histological staining techniques were used, each targeting specific cartilage and bone repair aspects. Hematoxylin-Eosin (H&E) staining provided a general assessment of tissue architecture, enabling the identification of cellular differentiation, inflammatory responses, and tissue structural integrity. Van Gieson staining was employed to evaluate the quality and organisation of the extracellular matrix, mainly focusing on collagen, a critical component for structural support and tissue resilience. Safranin-O staining, a well-established method in cartilage research, was used to detect proteoglycans essential components of the cartilage extracellular matrix that contribute to its compressive strength and elasticity. These staining methods are commonly applied in studies investigating the regenerative effects of exosomes on cartilage and bone tissues.

### Evaluated Parameters

Seven key parameters were analysed in histological sections:
1. **Cartilage edge regeneration:** Assessment of healing at the interface between healthy and damaged cartilage.
2. **Chondrocyte presence in transitional zones:** Evaluation of chondrocyte repopulation, essential for cartilage maintenance.
3. **Extracellular matrix regeneration (proteoglycans):** Quantifying proteoglycan deposition in the regenerated matrix.
4. **Subchondral bone vascularisation:** Observation of new blood vessel formation, crucial for bone repair.
5. **Trabecular bone regeneration:** Reconstruction of subchondral trabecular structures.
6. **Type II collagen formation:** The presence and organisation of type II collagen are hallmarks of hyaline cartilage regeneration.
7. Cartilage **fissures:** Analysis of the closure and repair of fissures in the regenerated cartilage.

### Evaluation Scale

Each parameter in this study was systematically evaluated using a standardised scoring system ranging from 0 to 3 to quantify the extent of tissue regeneration. A score of 0 indicated the complete absence of regeneration, representing no observable recovery or repair in the evaluated tissue. A score of 1 was assigned to areas demonstrating signs of initial regeneration, marked by minimal structural recovery or early-stage cellular activity. A score of 2 represented moderate regeneration, characterised by partial restoration of the tissue's structure and function but still falling short of complete repair. Lastly, a score of 3 denoted complete regeneration, where the tissue exhibited full structural and functional recovery, closely resembling healthy, uninjured tissue.

### Histological Assessment

Thirty-five tissue samples were collected and stained using the three selected techniques, resulting in 105 slides for analysis. Each slide underwent three independent evaluations by the same observer at different times to reduce intra-observer variability.

In total, 315 evaluations (105 slides × 3 readings) were performed to ensure consistent and robust data. Results were averaged when differences between readings were minor, while significant variations were resolved by considering all three observations. This rigorous process ensured reliable histological assessments, minimising subjective errors and capturing representative regenerative cartilage and subchondral bone changes.

### Results

### Descriptive Analysis of Experimental Groups

We evaluated the regenerative capacity of treatments applied to articular lesions in 35 lambs. The lesions simulated degenerative joint conditions and allowed a comparative assessment of four experimental groups: Control, Fresh Secretome, Cryopreserved Secretome, and Lyophilized Secretome groups. Seven key parameters were analysed across histological slides using repeated evaluations, revealing significant differences in regenerative outcomes among the treatments.

The Control group consistently showed minimal regeneration across most parameters, reflecting articular tissues' limited natural healing potential without therapeutic intervention. Conversely, groups treated with hAD-MSC-derived exosome concentrates particularly the Lyophilized group demonstrated notable improvements, with the Lyophilized group achieving the highest scores in several regenerative metrics.

### Comparison of Regenerative Parameters

Table 1 summarises the comparative analysis of regenerative parameters and provides an overview of the average scores for each treatment group across the key regenerative indicators.

**Table 1. Comparison of Regenerative Parameters**

| **Parameter** | **Control** | **Fresh** | **Cryopreserved** | **Lyophilised** |
|---|---|---|---|---|
| Cartilage Edge Regeneration | 1.33 | 1.50 | 1.50 | **2.33** |
| Chondrocyte Presence in Transitional Zones | 1.33 | 1.33 | 1.50 | **2.17** |
| Extracellular Matrix Regeneration (Proteoglycans) | 1.17 | 1.50 | 1.67 | **2.00** |
| Subchondral Bone Vascularization | 0.83 | 0.83 | 1.17 | **1.33** |
| Trabecular Bone Regeneration | 0.83 | 0.83 | 1.17 | **1.33** |
| Type II Collagen Formation | 1.33 | 1.50 | 1.17 | **1.83** |
| Cartilage Fissure Closure | 1.17 | 1.00 | 1.33 | **1.50** |

### 1. Cartilage Edge Regeneration

Cartilage regeneration was assessed using histological analysis with hematoxylin-eosin, Van Gieson's trichrome, and Safranin O staining, as shown in Figure 4. The control group (Fig. 4 A1, A5, A9) exhibited minimal evidence of cartilage regeneration, with pale pink staining using hematoxylin-eosin, faint reddish staining with Van Gieson's trichrome, and faint red staining with Safranin O, indicative of minimal extracellular matrix formation.

This was consistent with the limited regenerative capacity of cartilage, as reflected by an average score of 1.33.

The fresh group (Fig. 4 A2, A6, A10) demonstrated moderate regeneration, with more intense pink staining using hematoxylin-eosin, more marked reddish staining with Van Gieson's trichrome, and more evident reddish staining with Safranin O, indicating partial extracellular matrix organisation and proteoglycan presence. This group achieved an average score of 1.50.

The cryopreserved group (Fig. 4 A3, A7, A11) showed more prominent cartilage formation, with bright pink staining using hematoxylin-eosin, intense reddish staining with Van Gieson's trichrome, and significantly more uniform and intense red staining with Safranin O, which means advanced regeneration. This group also scored an average of 1.50, demonstrating improved matrix staining and tissue organisation compared to the fresh group.

Notably, the lyophilised group exhibited the most robust cartilage regeneration, achieving an average score of 2.33, indicative of near-complete regeneration in most specimens. Histological analysis (Fig. 4 A4, A8, A12) revealed a well-organized extracellular matrix with intense pink staining using hematoxylin-eosin, bright and homogenous reddish staining with Van Gieson's trichrome, and bright, uniform red staining with Safranin O, signifying abundant proteoglycans and active cartilage repair. The lyophilised group showed abundant, actively dividing chondrocytes and structurally complete tissue.

Overall, the results demonstrate a progression in cartilage regeneration from minimal in the control group to moderate in the fresh and cryopreserved groups, with the lyophilised group achieving the most significant and complete regeneration. These findings highlight lyophilised hAD-MSC-derived secretome containing exosomes as the most effective treatment option for cartilage repair.

### 2. Presence of Chondrocytes in Transitional Zones:

Chondrocytes, essential for maintaining cartilage health and the extracellular matrix, were more abundant in the transition areas of the lyophilised group. This group scored an average of 2.17, suggesting significant cellular repopulation at the interface between damaged and healthy tissue. The cryopreserved (1.50) and fresh groups (1.33) showed moderate chondrocyte presence, but their performance was notably lower than the lyophilised group. The control group, with a score of 1.33, further emphasised the importance of exosome treatments for promoting cellular regeneration in areas that otherwise lack the capacity for self-repair.

### 3. Extracellular Matrix (Proteoglycan) Regeneration:

Proteoglycans are vital for cartilage elasticity and strength. The lyophilised group achieved a near-complete restoration of the extracellular matrix, scoring an average of 2.00. This outcome highlights the ability of lyophilised exosomes to promote the formation of a functional cartilage matrix. The cryopreserved group also showed favourable results (1.67), underscoring its regenerative potential, though to a lesser extent than lyophilisation. The control group, with a low score of 1.17, reflected the insufficiency of natural cartilage repair processes in severe lesions.

### 4. Subchondral Bone Vascularization:

Histological evaluations of subchondral bone regeneration were performed using three staining methods: hematoxylin-eosin (A1-A4), Van Gieson's trichrome (A5-A8), and Safranin O (A9-A12), as shown in Figure 5.

The control group exhibited minimal bone regeneration, as evidenced by pale, poorly mineralised bone matrices and minimal osteoblast activity observed in hematoxylin-eosin staining. Bone lacunae appeared empty or poorly defined, while Van Gieson's trichrome staining showed weak reddish colouration, reflecting a low density of collagen fibres. Safranin O staining displayed an almost complete absence of colouration, indicating limited proteoglycan presence and minimal osteochondral regeneration. Additionally, angiogenesis in the control group was the lowest among all groups, with a score of 0.83, emphasising the limitations of natural healing and vascularisation in untreated lesions.

The fresh group demonstrated early signs of bone regeneration. Hematoxylin-eosin staining revealed increased osteoblast activity, with more intensely stained nuclei and signs of initial osteoid formation, visible as brighter pink areas in the trabeculae. Van Gieson's trichrome staining displayed a more uniform reddish colouration, indicating moderate increases in collagen fibre density and organisation. Safranin O staining showed improved colouration in transition zones between cartilage and bone, suggesting some proteoglycan production and matrix formation. Despite these improvements, angiogenesis in the fresh group remained limited, with a score of 0.83, lower than in the cryopreserved and lyophilised groups.

The cryopreserved group showed significant advancements in bone regeneration. Hematoxylin-eosin staining revealed a well-mineralized bone matrix with clearly defined and fully occupied bone lacunae and uniform osteoblast activity. Van Gieson's trichrome staining displayed intense and homogenous reddish colouration, reflecting a high density of organised collagen fibres. Safranin O staining showed stronger and more uniform colouration in the transition zones, indicating increased proteoglycan production and activity in osteochondral regions. Angiogenesis in this group was improved compared to the fresh group, with a score of 1.17, supporting enhanced vascularisation and nutrient delivery to the regenerating subchondral bone.

The group that received lyophilised treatment showed the strongest bone regeneration and vascularisation. Hematoxylin-eosin staining revealed compact trabeculae with uniform bright pink colouration and fully occupied bone lacunae, containing rows of active osteoblasts indicative of efficient and continuous bone remodelling. Van Gieson's trichrome staining showed the brightest and most uniform reddish colouration, reflecting high collagen density and alignment. Safranin O staining demonstrated bright and homogeneous red colouration, indicating advanced proteoglycan production and complete osteochondral integration. Angiogenesis in the lyophilised group was the most pronounced, with a score of 1.33, reflecting enhanced vascularisation and an improved supply of nutrients and oxygen to the regenerating tissue.

### Trabecular Bone Regeneration:

The restoration of trabecular bone is essential for subchondral structural integrity. The lyophilised group again performed the best, with compact trabeculae and a uniform bright pink colouration. Active osteoblasts occupied the bone lacunae, suggesting an efficient and continuous bone remodelling process. The cryopreserved group followed with moderate improvement (1.17), while the fresh and control groups showed limited results, underscoring the advantages of lyophilised exosomes for bone repair.

### 5. Type II Collagen Formation:

Type II collagen, a key component of hyaline cartilage, is critical for functional cartilage regeneration. The lyophilised group demonstrated superior performance in this parameter, with an average score of 1.83, indicating near-complete collagen formation. The cryopreserved (1.50) and fresh (1.17) groups showed intermediate results, while the control group exhibited minimal collagen formation, further emphasising the regenerative superiority of exosome treatments, particularly in their lyophilised form.

### 6. Cartilage Fissure Closure:

A critical measure of the quality and functionality of regenerated cartilage is the ability to close existing fissures. The lyophilised group showed the highest capacity for fissure closure, with most specimens exhibiting fully repaired cartilage. In contrast, the control and fresh groups exhibited open fissures, compromising the structural and functional integrity of the regenerated tissue.

An ANOVA analysis confirmed statistically significant differences in cartilage regeneration across groups (p = 0.0168). The Lyophilized group consistently showed superior outcomes in most parameters such as extracellular matrix regeneration, chondrocyte presence, and type II collagen formation, indicating that lyophilisation preserves the bioactive properties of hAD-MSC-derived secretome containing exosomes. Although parameters such as chondrocyte presence and bone regeneration did not reach statistical significance (p > 0.05), clear trends demonstrated the overall advantage of exosome treatments compared to natural healing.

The data also stressed the potential limitations of cryopreservation and fresh exosome preparations in achieving the regenerative capacity observed in the Lyophilized group. These findings highlight lyophilised hAD-MSC-derived secretome containing exosomes as the most effective treatment for articular repair, combining efficacy with practical storage and transport benefits.

### Example 2 - Quantitative proteomic analysis of human cell culture secretomes under different storage conditions

### METHODS

### a. Sample Reception

Twenty-four total samples were analized, corresponding to human cultured secretomes in different storage methods. The samples provided were: culture medium, fresh secretome, fresh concentrated secretome, cryopreserved (-80°C) secretome and lyophilized secretome. All samples were analyzed in triplicate.

### b. Protein Extraction

Proteins were extracted using 100 µl of lysis buffer (HEPES 50 mM, pH 8, Triton X-100 1% (wt/vol), NP-40 1% (v/v), Tween 20 1% (v/v), deoxycholate 1% (wt/v), EDTA 5 mM, NaCl 50 mM, glycerol 1% (v/v), 1X complete protease inhibitor and DTT 5 mM. The samples were incubated for half an hour at 60°C. Samples were then homogenised by ultrasound for 2 min with 10 sec cycles at 40% intensity, then alkylated using 20 mM iodoacetamide and 25 mM ammonium bicarbonate, and further incubated in the dark for 30 min. The protein extract was subjected to clean-up, using the chloroform/methanol method. One volume of the protein extract was added to 5 volumes of 100% methanol and one volume of 100% chloroform (v/v) was added. Three volumes of milliQ water were added, followed by centrifugation at 15,000 x g for 5 min, after which a protein disc was observed. The soluble phase was removed from the tube and the disc was washed with 400 µl of 100% methanol 4 times, and finally, dried in a rotary concentrator at 2,000 rpm overnight at 40 °C.

### c. Preparation for MS

The resulting proteins were precipitated by adding 5 volumes of cold acetone and incubated overnight at -80°C. They were then equilibrated at room temperature for 10 min, centrifuged at 16,000 x g for 15 min at 4°C and the supernatant was discarded. The resulting pellet was washed 3 times with cold 80% acetone. Subsequently, the protein pellet was allowed to dry in a rotary concentrator.

Samples were resuspended in 30 µL 8M Urea and 25 mM ammonium bicarbonate and then reduced with DTT to a final concentration of 20 mM in 25 mM ammonium bicarbonate and incubated for 1 hour at room temperature. After, the samples were alkylated by adding lodoacetamide to a final concentration of 20 mM in 25 mM ammonium bicarbonate and incubated for 1 hour in the dark at room temperature. Subsequently, the samples were diluted 8 times with 25 mM ammonium bicarbonate.

Digestion was performed with Trypsin sequencing grade (#V5071, Promega) in a 1:50 protease:protein ratio (mass/mass) and incubated for 16 h at 37°C. The digestion reaction was pH stopped by adding 10% formic acid. 200 ng of the peptides were cleaned up using Evotips disposable C18 columns (EVOSEP Biosystems) according to the supplier's instructions.

### d. LC-MS/MS (Liquid Chromatography - Tandem Mass Spectrometry)

Evotips columns were deposited on an Evosep One (Evosep Biosystems) coupled to timsTOF Pro 2 mass spectrometer ("Trapped Ion Mobility Spectrometry - Quadrupole Time Of Flight Mass Spectrometer", Bruker Daltonics) using an EVOSEP Performance column (15 cm x 150µm, 1.5µm beads ReproSil-Pur C18, EVOSEP Biosystems). Liquid chromatography was performed using the 30 SPD ("Samples per Day") mode on all samples. The gradient used was 2% to 35% buffer B (0.1% formic acid - acetonitrile). The collection of results was performed using TimsControl 2.0 software (Bruker Daltonics) under 10 cycles of PASEF, with a mass range of 100-1,700 m/z, capillary ionisation 1,500 V and a capillary temperature of 180°C, TOF frequency 10 KHz at a resolution of 50,000 FWHM.

### e. Protein identification

The obtained data were analysed with MSFragger v4.1 software (Kong et al., 2017, Nature Methods, 14(5), 513-520) via the Fragpipe v22.0 platform (https://fragpipe.nesvilab.org/) using the "default" workflow, on a data analysis server consisting of 48 cores and 512 Gb RAM. Precursor mass tolerance parameters of -20 to 20 PPM and mass fragment tolerance of 40 PPM were used. Digestion options included trypsin as enzyme, specific digestion mode and a maximum of 2 missed cleavages per peptide. Post-translational modifications (PTMs) used were: Carbamidomethylation of cysteine, as fixed PTMs: Methionine (M) oxidation, N-terminal acetylation, as variable PTMs. The database used for the identification was the proteome of *Homo sapiens* (code: UP000005640), available from Uniprot. Estimation of FDR <1% by means of a decoy database was included. Additionally, a database of common contaminants in mass spectrometry was added for protein identification analysis.

### f. Protein Quantification by Label Free Quantification

Label Free Quantification was performed by exporting and processing in the R statistical environment the reports of the identified reports. The intensity values for each run were normalised by adjusting the medians to a common value. Missing values were imputed within each category using the missForest algorithm (Stekhoven & Bühlmann, 2012, Bioinformatics, 28(1), 112-118), considering the presence of a protein in at least half of the replicates for each condition. To determine which proteins were differentially and significantly expressed in the treatment contrasts, the previously normalised data were fitted to a linear model and evaluated using a Bayes-based t-test. A protein was considered differentially expressed (DEP) if it had a p-value < 0.05.

### RESULTS

Cultured secretome samples, subjected to different storage methods, were processed for total protein extraction using a standard protocol described in the methodology. Data obtained from the mass spectrometer were analysed using FragPipe software, with the MSFragger search engine and the proteomes detailed in the protein identification section.

In total, 574 human proteins were identified across all experiments. Within the identification results, proteins present in the contaminant database integrated in the workflow were detected. The number of identified proteins per sample is presented in Table 2, together with the identifier and experimental condition.

The intensity matrix generated in FragPipe was subjected to quantification by Label-Free Quantification (LFQ) in the RStudio statistical environment. Table 3 shows the total number of proteins quantified and those differentially regulated (DRPs) in the comparisons performed. Table 4 shows the fold change of the proteins statistically significantly up regulated between the lyophilized secretome and the control.

**Table 2: Number of proteins obtained for each sample. Proteins identified according to the database are shown. Samples marked (*) were excluded for LFQ analysis.**

| **Sample label** | **Sample name** | **Condition** | **Replica** | **N° Peptides** | **N° Proteins** |
|---|---|---|---|---|---|
| **1** | White | WHITE | 1* | 984 | 257 |
| **2** | | | 2* | 770 | 222 |
| **3** | | | 3* | 701 | 227 |
| **4** | | | 4* | 676 | 203 |
| **5** | *Control* | CTRL | 1 | 3.240 | 487 |
| **6** | | | 2 | 3.392 | 507 |
| **7** | | | 3 | 3.147 | 429 |
| **8** | | | 4 | 3.435 | 532 |
| **9** | *Cryopreserved* | CRIO | 1 | 2.658 | 388 |
| **10** | | | 2 | 4.161 | 644 |
| **11** | | | 3 | 3.483 | 497 |
| **12** | | | 4 | 3.464 | 494 |
| **13** | *Filtering* | FILT | 1 | 3.612 | 457 |
| **14** | | | 2 | 3.473 | 476 |
| **15** | | | 3 | 3.442 | 451 |
| **16** | | | 4 | 3.142 | 405 |
| **17** | *Lyophilized* | LIOF | 1 | 3.889 | 540 |
| **18** | | | 2 | 4.365 | 606 |
| **19** | | | 3 | 4.084 | 530 |
| **20** | | | 4 | 2.373 | 378 |
| **21** | *Waste* | WASTE | 1* | 2.934 | 655 |
| **22** | | | 2* | 1.545 | 347 |
| **23** | | | 3* | 1.695 | 391 |
| **24** | | | 4* | 2.334 | 533 |

**Table 3: Total statistically significant differentially regulated proteins (DRPs) by comparison.**

| | **Proteins** | | |
|---|---|---|---|
| **Comparisons** | **Quantifiable** | **DEPs (P.value< 0.05)** | **DRPs combined** |
| **CRIO_vs_CTRL** | **574** | **49** | **222** |
| **FILT_vs_CTRL** | | **68** | |
| **LIOF_vs_CTRL** | | **129** | |
| **FILT_vs_CRIO** | | **40** | |
| **LIOF_vs_CRIO** | | **78** | |
| **LIOF_vs_FILT** | | **88** | |

**Table 4. Proteins statistically significantly up regulated in the liophylized secretome versus the fresh secretome. In bold are the proteins considered to be important for regenerative roles.**

| Protein | Log Fold Change |
|---|---|
| 1. Serine protease 1 | 3,605640443 |
| 2. Apolipoprotein A-1 | 3,161265362 |
| 3. Insulin | 3,12621989 |
| 4. Histone H2A type 2-C | 2,362072389 |
| 5. Polypeptide N-acetylgalactosaminyltransferase 2 | 2,273858346 |
| 6. Neuron-derived neurotrophic factor | 1,981869075 |
| 7. **Extracellular matrix protein 2** | 1,912050824 |
| 8. Semaphorin-7A | 1,866595373 |
| 9. A disintegrin and metalloproteinase with thrombospondin motifs 2 | 1,856155527 |
| 10. Actin, alpha cardiac muscle 1 | 1,723576546 |
| 11. Proteasome subunit alpha type-4 | 1,650095008 |
| 12. Thioredoxin domain-containing protein 5 | 1,55729331 |
| **13. Endoglin** | 1,48314076 |
| 14. N-acetylmuramoyl-L-alanine amidase | 1,474218957 |
| **15. Tissue factor pathway inhibitor** | 1,438182853 |
| 16. Pregnancy-specific beta-1-glycoprotein 3 | 1,416851619 |
| 17. Microfibril-associated glycoprotein 4 | 1,384859118 |
| 18. Neogenin | 1,376251612 |
| 19. Caldesmon | 1,358739595 |
| **20. Secreted frizzled-related protein 4** | 1,317055407 |
| 21. Calsyntenin-1 | 1,213756531 |
| 22. Complement factor H | 1,171852074 |
| 23. Renin receptor | 1,165544541 |
| 24. Beta-mannosidase | 1,154071858 |
| 25. Prelamin-A/C | 1,083212058 |
| 26. NPC intracellular cholesterol transporter 2 | 1,075705862 |
| 27. Vascular non-inflammatory molecule 2 | 1,068453721 |
| 28. Beta-galactosidase | 1,008120876 |
| **29. Midkine** | 0,996608566 |
| 30. Carboxypeptidase N catalytic chain | 0,994289404 |
| 31. Pregnancy zone protein | 0,973220093 |
| 32. Cathepsin C | 0,941116566 |
| **33. Dickkopf-related protein 3** | 0,93617855 |
| 34. Alpha-N-acetylgalactosaminidase | 0,916769286 |
| **35. Plasminogen** | 0,916470787 |
| 36. Cathepsin S | 0,912458154 |
| 37. Fructose-bisphosphate aldolase C | 0,862103978 |
| 38. Alpha-L-iduronidase | 0,851072342 |
| 39. Disintegrin and metalloproteinase domain-containing protein 10 | 0,724067129 |
| 40. Neutral alpha-glucosidase AB | 0,665270212 |
| 41. Thyroglobulin | 0,65828319 |
| 42. Keratin, type II cytoskeletal 1 | 0,62053424 |
| 43. Insulin-like growth factor-binding protein 6 | 0,597569966 |
| 44. Spondin-2 | 0,591893894 |
| **45. Prolargin** | 0,586716727 |
| 46. Peptidyl-glycine alpha-amidating monooxygenase | 0,565826805 |
| 47. Legumain | 0,563310024 |
| **48. Vitamin D-binding protein** | 0,562492838 |
| 49. Testican-1 | 0,545345553 |
| **50. Adipocyte enhancer-binding protein 1** | 0,532322728 |
| 51. Prosaposin | 0,429039615 |
| 52. Cathepsin K | 0,426167418 |
| 53. Amyloid-beta precursor protein | 0,399892246 |
| 54. Complement C3 | 0,383952498 |
| 55. Metalloproteinase inhibitor 1 | 0,383658348 |
| 56. Complement C1r subcomponent | 0,363063535 |
| 57. Prostaglandin-H2 D-isomerase | 0,335845535 |
| 58. Prothrombin | 0,308021909 |
| 59. Collagen alpha-1(III) chain | 0,299739899 |

## Claims

1. A lyophilized formulation comprising:
a) an adipose tissue mesenchymal stems cell (MSC) secretome, said secretome comprising:
(i) extracellular vesicle particles; and
(ii) the proteins extracellular matrix protein 2 (ECM2), Prolargin, Vitamin D-binding protein (DBP) and Midkine; and
b) a cryoprotectant, wherein said cryoprotectant is trehalose.

2. The lyophilized formulation according to claim 1, wherein the extracellular vesicle particles are exosomes.

3. The lyophilized formulation according to any one of claims 1 or 2, wherein the secretome further comprises one or more of the proteins selected from the group consisting of: Secreted frizzled-related protein 4 (SFRP4), Tissue factor pathway inhibitor (TFPI), Adipocyte enhancer-binding protein 1 (AEBP1), Dickkopf-related protein 3 (DKK3), Plasminogen, Endoglin and any combination thereof.

4. The lyophilized formulation according to any one of claims 1 to 3, wherein the concentration of trehalose before lyophilization is between 20 mM and 30 mM

5. The lyophilized formulation according to any one of claims 1 to 4, wherein the adipose tissue MSC secretome is a human adipose tissue MSC secretome.

6. The lyophilized formulation according to any one of claims 1 to 5, obtained by an *in vitro* method comprising the steps of:
i) providing adipose tissue comprising adipose tissue mesenchymal stromal cells;
ii) washing the adipose tissue of step i) obtaining a blood free lipid phase;
iii) separating the oil layer and the fat layer and discarding the oil layer obtaining a fat rich solution;
iv) digesting the collagen of the fat rich solution and discarding the oil layer obtaining a suspension;
v) washing and filtering the suspension to obtain a cell suspension;
vi) culturing the cell suspension to obtain a secretome;
vii) concentrating the secretome to obtain an exosome-rich secretome;
viii) adding trehalose to the exosome rich-secretome obtained in (vii); and
ix) lyophilizing the exosome rich-secretome obtained in (viii),
preferably wherein the *in vitro* method does not comprise a step of ultracentrifugation.

7. A pharmaceutical composition comprising the lyophilized formulation according to any one of claims 1 to 6 and a pharmaceutically acceptable diluent, preferably said composition is a reconstituted lyophilized injectable formulation with 2 ml of diluent.

8. The pharmaceutical composition according to claim 7, wherein the average particle size is of 167.6 ± 9.2 nm and/or wherein the concentration of particles is of 3.70 × 10¹ ± 2.31 × 10⁸ particles/ml.

9. The lyophilized formulation according to any one of claims 1 to 6 or the pharmaceutical composition according to any one of claims 7 to 8 for use in medicine, preferably in regenerative medicine.

10. The lyophilized formulation according to any one of claims 1 to 6 or the pharmaceutical composition according to any one of claims 7 to 8 for use in the prevention and/or treatment of a joint lesion or a degenerative joint disease.

11. The lyophilized formulation or the pharmaceutical composition for use according to claim 10, wherein the degenerative joint disease is osteoarthritis.

12. The lyophilized formulation or pharmaceutical composition for use according to any one of claims 9 to 11, wherein the administration is done by intra-articular injection, preferably at the lesion site, more preferably at the suprapatellar bursa.

13. The lyophilized formulation or pharmaceutical composition for use according to any one of claims 9 to 12, wherein the administration is done with a 2 ml reconstituted lyophilized formulation.

14. The lyophilized formulation or pharmaceutical composition for use according to any one of claims 9 to 13, wherein the administration is done one, two, three, four or more times, and wherein the second administration is done 7 days after the first one, and the third and fourth administrations are done at 15 days interval from the previous administration.

15. A kit comprising the lyophilized formulation according to any one of claims 1 to 6 and a pharmaceutically acceptable diluent.
